# EUROPEAN PATENT SPECIFICATION

(11) **EP 3 759 492 B1**
(45) Date of publication and mention of the grant of the patent: **11.11.2026**
(21) Application number: 19708997.2
(22) Date of filing: 27.02.2019
(51) Int. Cl.: C07K 16/26, G01N 33/575

(54) **PROGASTRIN AS A BIOMARKER FOR IMMUNOTHERAPY**
PROGASTRIN ALS BIOMARKER FÜR IMMUNTHERAPIE
PROGASTRINE EN TANT QUE BIOMARQUEUR POUR L'IMMUNOTHÉRAPIE

(30) Priority: 27.02.2018 US 201862635620 P
(43) Date of publication of application: 06.01.2021
(73) Proprietor: ECS-Progastrin SA, 1008 Prilly (CH)
(72) Inventor: JOUBERT, Dominique, 34200 Sète (FR)
(74) Representative: Regimbeau
(86) International application number: PCT/EP2019/054878
(87) International publication number: WO 2019/166499

(56) References cited:
- WO-A1-2006/032980
- WO-A1-2011/083089
- WO-A1-2017/114973
- WO-A1-2017/114975
- WO-A1-2018/028449
- WO-A2-2011/045080

## Description

### INTRODUCTION

Immunotherapy has been a game-changer in the field of cancer therapy. Developments in immune checkpoint-based therapy are progressing at a breathtaking pace. In order to ensure that an immune inflammatory response is not constantly activated once tumour antigens have stimulated a response, multiple controls or "checkpoints" are in place or activated. These checkpoints are mostly represented by T-cell receptor binding to ligands on cells in the surrounding tumour microenvironment, forming immunological synapses which then regulate the function of the T cell.

Despite the promise of immunotherapy for treating advanced cancers, a number of challenges remain. Typically, only a small fraction of patients achieves durable long-lasting responses to therapy. Further, measuring tumour responses is complicated by the fact that responding patients may initially experience an increase in tumour size or seemingly develop new lesions on radio-graphic images.

A particular challenge in cancer immunotherapy has been the identification of mechanism-based biomarkers that could be used to select candidates for such treatment and guide disease-management decisions (Topalian et al., N Engl J Med, 366(26): 2443-54 (2012)). Therefore, there is a critical need for standardised and validated biomarkers that yield actionable insights into immunotherapy efficacy at every stage of cancer development. In addition to helping identify patients who could benefit from available therapies, biomarkers may be useful for monitoring treatment response. These indicators also have the potential to shed light on a treatment's mechanism of action, which would provide important insight for optimising treatment approaches and defining rational combination therapies. However, the intrinsic characteristics of malignant tumours-such as their heterogeneity, plasticity, and diversity-pose challenges to biomarker development.

WO 2018/028449 discloses methods of predicting the efficacy of immune checkpoint inhibitor therapy in cancer patients.

Genetic mutations are a hallmark of malignant tumours and are responsible for the vast majority of cancer's life-threatening characteristics, such as ceaseless growth and metastasis, or spreading within the body. Some of these mutations are associated with the response to immune checkpoint inhibitors. The number of mutations that a tumour has accumulated, referred to as tumour mutational burden (TMB), is itself a biomarker. Recently, it has been shown that the response to immunotherapy is determined by the composition of gut microbiota. These features have been used to design biomarkers to prognose the outcome of immunotherapy which are mostly genetic (Yan et al., Front Pharmacol. 9: 1050 2018). However, the use of such biomarkers requires next-generation sequencing, which can be difficult to use routinely in a clinical lab. Thus, there is still a need for biomarkers which can be used easily and reliably to predict the patient's response to immunotherapy.

### SUMMARY OF DESCRIPTION

The invention is defined in the appended claims and any other aspects or embodiments set forth herein are for information only. In particular, references to the methods of treatment by therapy or in vivo diagnosis methods in this description are to be interpreted as references to the antibodies, pharmaceutical compositions and medicaments of the present invention for use in those methods.

The present disclosure is related to the discovery that levels of certain biomarkers, including progastrin, in the fluids of cancer patients are negative predictors of those patients who will respond to treatment with immune checkpoint inhibitors.

More specifically, the invention disclosed herein relates to an in vitro method for selecting a melanoma patient susceptible to responding to treatment with an immune checkpoint inhibitor, said method comprising the steps of:
a) contacting a biological sample from said subject with at least one progastrin-binding monoclonal antibody,
   wherein the biological sample is blood, plasma or serum,
   and wherein the monoclonal antibody is selected in the group consisting of:
      - A monoclonal antibody comprising a heavy chain comprising CDR-H1, CDR-H2 and CDR-H3 of amino acid sequences SEQ ID N°4, 5 and 6, respectively, and a light chain comprising CDR-L1, CDR-L2 and CDR-L3 of amino acid sequences SEQ ID N°7, 8 and 9, respectively,
      - A monoclonal antibody comprising a heavy chain comprising CDR-H1, CDR-H2 and CDR-H3 of amino acid sequences SEQ ID N°10, 11 and 12, respectively, and a light chain comprising CDR-L1, CDR-L2 and CDR-L3 of amino acid sequences SEQ ID N°13, 14 and 15, respectively,
      - A monoclonal antibody comprising a heavy chain comprising CDR-H1, CDR-H2 and CDR-H3 of amino acid sequences SEQ ID N°16, 17 and 18, respectively, and a light chain comprising CDR-L1, CDR-L2 and CDR-L3 of amino acid sequences SEQ ID N°19, 20 and 21, respectively,
      - A monoclonal antibody comprising a heavy chain comprising CDR-H1, CDR-H2 and CDR-H3 of amino acid sequences SEQ ID N°22, 23 and 24, respectively, and a light chain comprising CDR-L1, CDR-L2 and CDR-L3 of amino acid sequences SEQ ID N°25, 26 and 27, respectively,
      - A monoclonal antibody comprising a heavy chain comprising CDR-H1, CDR-H2 and CDR-H3 of amino acid sequences SEQ ID N°28, 29 and 30, respectively, and a light chain comprising CDR-L1, CDR-L2 and CDR-L3 of amino acid sequences SEQ ID N°31, 32 and 33, respectively,
      - A monoclonal antibody comprising a heavy chain comprising CDR-H1, CDR-H2 and CDR-H3 of amino acid sequences SEQ ID N°34, 35 and 36, respectively, and a light chain comprising CDR-L1, CDR-L2 and CDR-L3 of amino acid sequences SEQ ID N°37, 38 and 39, respectively, and
      - A monoclonal antibody produced by the hybridoma deposited at the CNCM, Institut Pasteur, 25-28 rue du Docteur Roux, 75724 Paris CEDEX 15, France, on 27 December 2016, under reference I-5158.
         and
b) detecting the binding of said progastrin-binding molecule to progastrin in said sample, wherein a concentration of progastrin of at least 3 pM in said biological sample is indicative of the presence of a cancer which is not responsive to treatment with an immune checkpoint inhibitor in said subject.

Preferably, the method comprises the further steps of:
c) determining a reference concentration of progastrin in a reference sample,
d) comparing the concentration of progastrin in said biological sample with said reference concentration of progastrin,
e) determining, from the comparison of step d), whether said patient is responsive or not to treatment with an immune checkpoint inhibitor.

In an embodiment, the determination of step a) includes:
(i) contacting said sample with a first progastrin-binding antibody which binds to a first epitope of progastrin, and
(ii) contacting said sample with a second progastrin-binding antibody which binds to a second epitope of progastrin.

In an embodiment, the first progastrin-binding antibody binds an epitope within the C-terminus of progastrin. Preferably, the progastrin-binding antibody is a monoclonal antibody produced by the hybridoma deposited at the CNCM, Institut Pasteur, 25-28 rue du Docteur Roux, 75724 Paris CEDEX 15, France, on 27 December 2016, under reference I-5158.

In an embodiment, the second progastrin-binding antibody binds an epitope within the N-terminus of progastrin. Preferably, the second progastrin-binding antibody is:
- a polyclonal antibody binding an epitope within the N-terminus of progastrin or
- a monoclonal antibody comprising a heavy chain comprising the following three CDRs, CDR-H1, CDR-H2 and CDR-H3 of amino acid sequences SEQ ID N°16, 17 and 18, respectively, and a light chain comprising the following three CDRs, CDR-L1, CDR-L2 and CDR-L3 of amino acid sequences SEQ ID N°19, 20 and 21, respectively.

In an embodiment, the level of progastrin is determined in step a) with an ELISA.

In an embodiment, the biological sample is contacted with a first antibody, which binds to a first part of progastrin, and with a second antibody, which binds to a second part of progastrin.

In another aspect, the invention also relates to an immune checkpoint inhibitor for use in treating cancer, said use comprising a prior step of:
a) selecting a patient responsive to immune checkpoint inhibitors using a method disclosed herein.

In yet another aspect, the invention relates to an in vitro method for prognosing a cancer treatment with an immune checkpoint inhibitor in a subject, said method comprising the steps of:
a) contacting a biological sample from said subject with at least one progastrin-binding monoclonal antibody,
   wherein the biological sample is blood, plasma or serum,
   and wherein the monoclonal antibody is selected in the group consisting of:
      - A monoclonal antibody comprising a heavy chain comprising CDR-H1, CDR-H2 and CDR-H3 of amino acid sequences SEQ ID N°4, 5 and 6, respectively, and a light chain comprising CDR-L1, CDR-L2 and CDR-L3 of amino acid sequences SEQ ID N°7, 8 and 9, respectively,
      - A monoclonal antibody comprising a heavy chain comprising CDR-H1, CDR-H2 and CDR-H3 of amino acid sequences SEQ ID N°10, 11 and 12, respectively, and a light chain comprising CDR-L1, CDR-L2 and CDR-L3 of amino acid sequences SEQ ID N°13, 14 and 15, respectively,
      - A monoclonal antibody comprising a heavy chain comprising CDR-H1, CDR-H2 and CDR-H3 of amino acid sequences SEQ ID N°16, 17 and 18, respectively, and a light chain comprising CDR-L1, CDR-L2 and CDR-L3 of amino acid sequences SEQ ID N°19, 20 and 21, respectively,
      - A monoclonal antibody comprising a heavy chain comprising CDR-H1, CDR-H2 and CDR-H3 of amino acid sequences SEQ ID N°22, 23 and 24, respectively, and a light chain comprising CDR-L1, CDR-L2 and CDR-L3 of amino acid sequences SEQ ID N°25, 26 and 27, respectively,
      - A monoclonal antibody comprising a heavy chain comprising CDR-H1, CDR-H2 and CDR-H3 of amino acid sequences SEQ ID N°28, 29 and 30, respectively, and a light chain comprising CDR-L1, CDR-L2 and CDR-L3 of amino acid sequences SEQ ID N°31, 32 and 33, respectively,
      - A monoclonal antibody comprising a heavy chain comprising CDR-H1, CDR-H2 and CDR-H3 of amino acid sequences SEQ ID N°34, 35 and 36, respectively, and a light chain comprising CDR-L1, CDR-L2 and CDR-L3 of amino acid sequences SEQ ID N°37, 38 and 39, respectively, and
      - A monoclonal antibody produced by the hybridoma deposited at the CNCM, Institut Pasteur, 25-28 rue du Docteur Roux, 75724 Paris CEDEX 15, France, on 27 December 2016, under reference I-5158.
         and
b) detecting the binding of said progastrin-binding molecule to progastrin in said sample, wherein a concentration of progastrin of at least 3 pM in said biological sample indicates the prognosis is negative.

Preferably, the method comprises the further steps of:
a) determining a reference concentration of progastrin in a reference sample,
b) comparing the concentration of progastrin in said biological sample with said reference concentration of progastrin,
c) prognosing, from the comparison of step d), said cancer treatment with an immune checkpoint inhibitor.

Accordingly, in a first aspect, a method is herein provided for selecting a cancer patient having an immune-checkpoint inhibitor responsive or non-responsive phenotype. This method comprises detecting the binding of a progastrin-binding molecule to a biological sample of said patient, wherein said binding indicates that the patient will not respond to treatment with an immune checkpoint inhibitor and thus have an immune-checkpoint inhibitor non-responsive phenotype.

In another aspect of the present disclosure, a method is provided for the in vitro diagnosis of a cancer which is not susceptible to treatment with an immune checkpoint inhibitor in a patient. In other words, said cancer is not responsive to a treatment with an immune checkpoint inhibitor. According to this method, the binding of a progastrin-molecule to a biological sample of said patient indicates that that the cancer will not respond to said treatment.

Another method provided herein relates to the in vitro diagnosis of a metastasised cancer which is not susceptible to treatment with an immune checkpoint inhibitor in a patient. In other words, said metastasised cancer is not responsive to a treatment with an immune checkpoint inhibitor. According to this method, the binding of a progastrin-molecule to a biological sample of said patient indicates that the metastasised cancer will not respond to said treatment.

In another aspect, the present disclosure relates to a method of the in vitro prognosis of a cancer treatment with an immune checkpoint inhibitor in a patient. This method comprises a step of detecting the binding of a progastrin-binding molecule to a biological sample of said patient, wherein said binding indicates a negative prognosis.

Preferably, the levels of progastrin in said sample are measured. A concentration of progastrin of at least 3 pM, at least 5 pM, at least 10 pM, at least 20 pM, at least 30 pM, in said biological sample indicates that the treatment with an immune checkpoint inhibitor will not lead to a significant response.

Another aspect relates to a method of treating cancer with an immune checkpoint inhibitor. A method for designing a treatment of cancer with an immune checkpoint inhibitor is also provided, in another aspect. Said methods both comprise a prior step of selecting a patient responsive to immune checkpoint inhibitors by any of the methods described above.

The present disclosure also provides a method of adapting a treatment of cancer in a patient with an immune checkpoint inhibitor. This method also comprises a prior step of assaying the immune-checkpoint-inhibitors responsive or non-responsive phenotype of the patient by any of the methods described above. Said adaptation of the immune-checkpoint-inhibitor treatment may consist in a reduction or suppression of said treatment if the patient's phenotype is non-responsive, or, alternatively, the continuation of said treatment if said phenotype is responsive.

All methods and materials similar or equivalent to those described herein can be used in the practice or testing of the present disclosure, with suitable methods and materials being described herein. The practice of the disclosure employs, unless other otherwise indicated, conventional techniques or protein chemistry, molecular virology, microbiology, recombinant DNA technology, and pharmacology, which are within the skill of the art. Such techniques are explained fully in the literature (see e.g., Ausubel et al., Short Protocols in Molecular Biology, Current Protocols; 5th Ed., 2002; Remington's Pharmaceutical Sciences, 17th ed., Mack Publishing Co., Easton, Pa., 1985; and Sambrook et al., Molecular Cloning: A Laboratory Manual, Cold Spring Harbor Laboratory Press; 3rd Ed., 2001). The nomenclatures used in connection with, and the laboratory procedures and techniques of, molecular and cellular biology, protein biochemistry, enzymology and medicinal and pharmaceutical chemistry described herein are those well-known and commonly used in the art.

### LEGENDS OF FIGURES

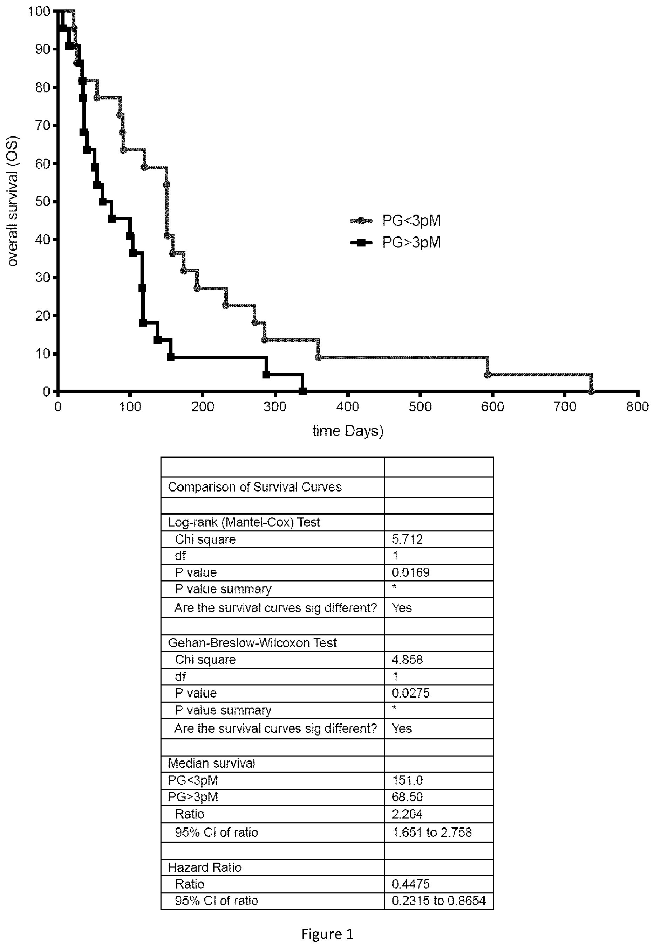

**Figure 1****:** Overall survival of melanoma patients treated with immunotherapy. PG levels were measured before treatment. Only patients who died are included in the study.

### DETAILED DESCRIPTION

### Definitions

Unless specifically defined, all technical and scientific terms used herein have the same meaning as commonly understood by a skill artisan in chemistry, biochemistry, cellular biology, molecular biology, and medical sciences.

The term "about" or "approximately" refers to the normal range of error for a given value or range known to the person of skills in the art. It usually means within 20%, such as within 10%, or within 5% (or 1% or less) of a given value or range.

As used herein, "administer" or "administration" refers to the act of injecting or otherwise physically delivering a substance as it exists outside the body (e.g., an anti-progastrin antibody provided herein) into a patient, such as by mucosal, intradermal, intravenous, intramuscular delivery and/or any other method of physical delivery described herein or known in the art. When a disease, or a symptom thereof, is being treated, administration of the substance typically occurs after the onset of the disease or symptoms thereof. When a disease, or symptoms thereof, are being prevented, administration of the substance typically occurs before the onset of the disease or symptoms thereof.

The terms "antibody" and "immunoglobulin" or "Ig" are used interchangeably herein. These terms are used herein in the broadest sense and specifically cover monoclonal antibodies (including full length monoclonal antibodies) of any isotype such as IgG, IgM, IgA, IgD, and IgE, polyclonal antibodies, multispecific antibodies, chimeric antibodies, and antibody fragments, provided that said fragments retain the desired biological function. These terms are intended to include a polypeptide product of B cells within the immunoglobulin class of polypeptides that is capable of binding to a specific molecular antigen and is composed of two identical pairs of polypeptide chains inter-connected by disulphide bonds, wherein each pair has one heavy chain (about 50-70 kDa) and one light chain (about 25 kDa) and each amino-terminal portion of each chain includes a variable region of about 100 to about 130 or more amino acids and each carboxy-terminal portion of each chain includes a constant region (See, Borrebaeck (ed.) (1995) Antibody Engineering, Second Ed., Oxford University Press.; Kuby (1997) Immunology, Third Ed., W.H. Freeman and Company, New York). Each variable region of each heavy and light chain is composed of three complementarity-determining regions (CDRs), which are also known as hypervariable regions and four frameworks (FRs), the more highly conserved portions of variable domains, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g. effector cells) and the first component (C1q) of the classical complement system. In some instances, the specific molecular antigen can be bound by an antibody provided herein includes the target progastrin polypeptide, fragment or epitope. An antibody reactive with a specific antigen can be generated by recombinant methods such as selection of libraries of recombinant antibodies in phage or similar vectors, or by immunising an animal with the antigen or an antigen-encoding nucleic acid.

Antibodies also include, but are not limited to, synthetic antibodies, monoclonal antibodies, recombinantly produced antibodies, multispecific antibodies (including bi-specific antibodies), human antibodies, humanised antibodies, camelised antibodies, chimeric antibodies, intrabodies, anti-idiotypic (anti-Id) antibodies, and functional fragments of any of the above, which refers a portion of an antibody heavy or light chain polypeptide that retains some or all of the biological function of the antibody from which the fragment was derived. The antibodies provided herein can be of any type (*e.g.,* IgG, IgE, IgM, IgD, IgA and IgY), any class (*e.g.,* IgG1, IgG2, IgG3, IgG4, IgA1 and IgA2), or any subclass (e.g., IgG2a and IgG2b) of immunoglobulin molecule.

The terms "anti-progastrin antibodies," "antibodies that bind to progastrin," "antibodies that bind to a progastrin epitope," and analogous terms are used interchangeably herein and refer to antibodies that bind to a progastrin polypeptide, such as a progastrin antigen or epitope. Such antibodies include polyclonal and monoclonal antibodies, including chimeric and humanised antibodies. An antibody that binds to a progastrin antigen may be cross-reactive with related antigens. In some instances, an antibody that binds to progastrin does not cross-react with other antigens such as e.g., other peptides or polypeptides derived from the gastrin gene. An antibody that binds to progastrin can be identified, for example, by immunoassays, BIAcore, or other techniques known to those of skill in the art. An antibody binds to progastrin, for example, when it binds to progastrin with higher affinity than to any cross-reactive antigen as determined using experimental techniques, such as radioimmunoassays (RIA) and enzyme-linked immunosorbent assays (ELISAs), for example, an antibody that specifically binds to progastrin. Typically, a specific or selective reaction will be at least twice background signal or noise and may be more than 10 times background. See, e.g., Paul, ed., 1989, Fundamental Immunology Second Edition, Raven Press, New York at pages 332-336 for a discussion regarding antibody specificity. In some instances, an antibody "which binds" an antigen of interest is one that binds the antigen with sufficient affinity such that the antibody is useful as a diagnostic and/or therapeutic agent in targeting a cell or tissue expressing the antigen, and does not significantly cross-react with other proteins. In such instances, the extent of binding of the antibody to a "non-target" protein will be less than about 10% of the binding of the antibody to its particular target protein as determined by fluorescence activated cell sorting (FACS) analysis or radioimmunoprecipitation (RIPA). With regard to the binding of an antibody to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labelled target. In this case, specific binding is indicated if the binding of the labelled target to a probe is competitively inhibited by excess unlabelled target. The term "specific binding" or "specifically binds to" or is "specific for" a particular polypeptide or an epitope on a particular polypeptide target as used herein can be exhibited, for example, by a molecule having a K_{D} for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. In some instances, the term "specific binding" refers to binding where a molecule binds to a particular polypeptide or epitope on a particular polypeptide without substantially binding to any other polypeptide or polypeptide epitope. In some instances, an antibody that binds to progastrin has a dissociation constant (K_{D}) of ≤ 1µM, ≤ 100 nM, ≤ 10 nM, ≤ 1nM, or ≤ 0.1nM.

As used herein, the term "antigen" refers to a predetermined antigen to which an antibody can selectively bind. The target antigen may be a polypeptide, carbohydrate, nucleic acid, lipid, hapten or other naturally occurring or synthetic compound. Preferably, the target antigen is a polypeptide, including, for example, a progastrin polypeptide.

The term "antigen binding fragment," "antigen binding domain," "antigen binding region," and similar terms refer to that portion of an antibody which comprises the amino acid residues that interact with an antigen and confer on the binding agent its specificity and affinity for the antigen (e.g., the complementarity determining regions (CDRs)). By the expression "antigen-binding fragment" of an antibody, it is intended to indicate any peptide, polypeptide, or protein retaining the ability to bind to the target (also generally referred to as antigen) of the said antibody, generally the same epitope, and comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, or at least 200 contiguous amino acid residues, of the amino acid sequence of the antibody. More particularly, the said antigen-binding fragment comprises at least one CDR of the antibody from which it is derived. More preferably, the said antigen binding fragment comprises 2, 3, 4 or 5 CDRs, more preferably the 6 CDRs of the antibody from which it is derived.

The "antigen-binding fragments" can be selected, without limitation, in the group consisting of Fab, Fab', (Fab')₂, Fv, scFv (sc for single chain), Bis-scFv, scFv-Fc fragments, Fab2, Fab3, minibodies, diabodies, triabodies, tetrabodies, and nanobodies, and fusion proteins with disordered peptides such as XTEN (extended recombinant polypeptide) or PAS motifs, and any fragment of which the half-life time would be increased by chemical modification, such as the addition of poly(alkylene) glycol such as poly(ethylene) glycol ("PEGylation") (pegylated fragments called Fv-PEG, scFv-PEG, Fab-PEG, F(ab')₂-PEG or Fab'-PEG) ("PEG" for Poly(Ethylene) Glycol), or by incorporation in a liposome, said fragments having at least one of the characteristic CDRs of the antibody disclosed herein. Preferably, said "antigen-binding fragments" will be constituted or will comprise a partial sequence of the heavy or light variable chain of the antibody from which they are derived, said partial sequence being sufficient to retain the same specificity of binding as the antibody from which it is descended and a sufficient affinity, preferably at least equal to 1/100, in a more preferred manner to at least 1/10, of the affinity of the antibody from which it is descended, with respect to the target. Such antibody fragments can be found described in, for example, Harlow and Lane, Antibodies: A Laboratory Manual, Cold Spring Harbor Laboratory, New York (1989); Myers (ed.), Molec. Biology and Biotechnology: A Comprehensive Desk Reference, New York: VCH Publisher, Inc.; Huston et al., Cell Biophysics, 22:189-224 (1993); Plückthun and Skerra, Meth. Enzymol., 178:497-515 (1989) and in Day, E.D., Advanced Immunochemistry, Second Ed., Wiley-Liss, Inc., New York, NY (1990).

The terms "binds" or "binding" as used herein refer to an interaction between molecules to form a complex which, under physiologic conditions, is relatively stable. Interactions can be, for example, non-covalent interactions including hydrogen bonds, ionic bonds, hydrophobic interactions, and/or van der Waals interactions. A complex can also include the binding of two or more molecules held together by covalent or non-covalent bonds, interactions or forces. The strength of the total non-covalent interactions between a single antigen-binding site on an antibody and a single epitope of a target molecule, such as progastrin, is the affinity of the antibody or functional fragment for that epitope. The ratio of association (*k₁*) to dissociation (*k₋₁*) of an antibody to a monovalent antigen (*k₁*/ *k₋₁*) is the association constant K, which is a measure of affinity. The value of K varies for different complexes of antibody and antigen and depends on both *k₁* and *k₋₁*. The association constant K for an antibody provided herein can be determined using any method provided herein or any other method well known to those skilled in the art. The affinity at one binding site does not always reflect the true strength of the interaction between an antibody and an antigen. When complex antigens containing multiple, repeating antigenic determinants, such as a polyvalent progastrin, come in contact with antibodies containing multiple binding sites, the interaction of antibody with antigen at one site will increase the probability of a reaction at a second site. The strength of such multiple interactions between a multivalent antibody and antigen is called the avidity. The avidity of an antibody can be a better measure of its binding capacity than is the affinity of its individual binding sites. For example, high avidity can compensate for low affinity as is sometimes found for pentameric IgM antibodies, which can have a lower affinity than IgG, but the high avidity of IgM, resulting from its multivalence, enables it to bind antigen effectively. Methods for determining whether two molecules bind are well known in the art and include, for example, equilibrium dialysis, surface plasmon resonance, and the like. In particular, said antibody, or antigen-binding fragment thereof, binds to progastrin with an affinity that is at least two-fold greater than its affinity for binding to a non-specific molecule such as BSA or casein. More particularly, said antibody, or antigen-binding fragment thereof, binds only to progastrin.

As used herein, the term "biological sample" or "sample" refers to a sample that has been obtained from a biological source, such as a patient or subject. A "biological sample" as used herein refers notably to a whole organism or a subset of its tissues, cells or component parts (e.g. blood vessel, including artery, vein and capillary, body fluids, including but not limited to blood, serum, mucus, lymphatic fluid, synovial fluid, cerebrospinal fluid, saliva, amniotic fluid, amniotic cord blood, urine, vaginal fluid and semen). "Biological sample" further refers to a homogenate, lysate or extract prepared from a whole organism or a subset of its tissues, cells or component parts, or a fraction or portion thereof. Lastly, "biological sample" refers to a medium, such as a nutrient broth or gel in which an organism has been propagated, which contains cellular components, such as proteins or nucleic acid molecules.

As used herein, the term "biomarker" is intended to encompass a biochemical characteristic that is used as an indicator of a biologic state and includes genes (and nucleotide sequences of such genes), mRNAs (and nucleotide sequences of such mRNAs) and proteins (and amino acid sequences of such proteins) and post-translationally modified forms of proteins (i.e. phosphorylated and non-phosphorylated forms). A biomarker may notably refer to a substance that can be used to diagnose, or to measure the progress of a disease or condition, or the effects of treatment of a disease or condition is meant. A biomarker can be, for example, the presence of a nucleic acid, protein, or antibody associated with the presence of cancer or another disease in an individual. A "biomarker expression pattern" is intended to refer to a quantitative or qualitative summary of the expression of one or more biomarkers in a subject, such as in comparison to a standard or a control.

The term "block," or a grammatical equivalent thereof, when used in the context of an antibody refers to an antibody that prevents or stops a biological activity of the antigen to which the antibody binds. A blocking antibody includes an antibody that combines with an antigen without eliciting a reaction, but that blocks another protein from later combining or complexing with that antigen. The blocking effect of an antibody can be one which results in a measurable change in the antigen's biological activity.

The terms "cell proliferative disorder" and "proliferative disorder" refer to disorders that are associated with some degree of abnormal cell proliferation. The cell proliferative disorder can be for example a tumour or cancer. "Tumour," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumour" are not mutually exclusive as referred to herein. The terms "cancer" and "cancerous" refer to or describe the physiological condition in mammals that is typically characterised by unregulated cell growth. A "cancer" as used herein is any malignant neoplasm resulting from the undesired growth, the invasion, and under certain conditions metastasis of impaired cells in an organism. The cells giving rise to cancer are genetically impaired and have usually lost their ability to control cell division, cell migration behaviour, differentiation status and/or cell death machinery. Most cancers form a tumour but some hematopoietic cancers, such as leukaemia, do not. Thus, a "cancer" as used herein may include both benign and malignant cancers.

A "chemotherapeutic agent" is a chemical or biological agent (e.g., an agent, including a small molecule drug or biologic, such as an antibody or cell) useful in the treatment of cancer, regardless of mechanism of action. Chemotherapeutic agents include compounds used in targeted therapy and conventional chemotherapy. Chemotherapeutic agents include, but are not limited to, alkylating agents, antimetabolites, anti-tumour antibiotics, mitotic inhibitors, chromatin function inhibitors, anti-angiogenesis agents, anti-oestrogens, anti-androgens or immunomodulators.

The term "chimeric" antibody refers to an antibody in which a portion of the heavy and/or light chain is derived from a particular source or species, while the remainder of the heavy and/or light chain is derived from a different source or species. In an instance, a "chimeric antibody" is an antibody in which the constant region, or a portion thereof, is altered, replaced, or exchanged, so that the variable region is linked to a constant region of a different species, or belonging to another antibody class or subclass. In another instance, a "chimeric antibody" refers to an antibody in which the variable region, or a portion thereof, is altered, replaced, or exchanged, so that the constant region is linked to a variable region of a different species, or belonging to another antibody class or subclass.

As used herein, a "CDR" refers to one of three hypervariable regions (H1, H2 or H3) within the non-framework region of the immunoglobulin (Ig or antibody) VH β-sheet framework, or one of three hypervariable regions (L1, L2 or L3) within the non-framework region of the antibody VL β-sheet framework. Accordingly, CDRs are variable region sequences interspersed within the framework region sequences. CDR regions are well known to those skilled in the art and have been defined by, for example, Kabat as the regions of most hypervariability within the antibody variable (V) domains (Kabat et al., J. Biol. Chem. 252:6609-6616 (1977); Kabat, Adv. Prot. Chem. 32:1-75 (1978)). The Kabat CDRs are based on sequence variability and are the most commonly used (Kabat etal., Sequences of Proteins of Immunological Interest, 5th Ed. Public Health Service, National Institutes of Health, Bethesda, MD. (1991)). Chothia refers instead to the location of the structural loops (Chothia and Lesk J Mol. Bioi. 196:901-917 (1987)). CDR region sequences also have been defined structurally by Chothia as those residues that are not part of the conserved β-sheet framework, and thus are able to adopt different conformations (Chothia and Lesk, J. Mol. Biol. 196:901-917 (1987)). The end of the Chothia CDR-H1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). Both terminologies are well recognised in the art. CDR region sequences have also been defined by AbM, Contact and IMGT. The AbM hypervariable regions represent a compromise between the Kabat CDRs and Chothia structural loops, and are used by Oxford Molecular's AbM antibody modelling software. The "contact" hypervariable regions are based on an analysis of the available complex crystal structures. Recently, a universal numbering system has been developed and widely adopted, ImMunoGeneTics (IMGT) Information System^{®} (Lafranc et al., Dev. Comp. Immunol. 27(1):55-77 (2003)). The IMGT universal numbering has been defined to compare the variable domains whatever the antigen receptor, the chain type, or the species [Lefranc M.-P., Immunology Today 18, 509 (1997) / Lefranc M.-P., The Immunologist, 7, 132-136 (1999)]. In the IMGT universal numbering, the conserved amino acids always have the same position, for instance cysteine 23 (1st-CYS), tryptophan 41 (CONSERVED-TRP), hydrophobic amino acid 89, cysteine 104 (2nd-CYS), phenylalanine or tryptophan 118 (J-PHE or J-TRP). The IMGT universal numbering provides a standardised delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. As gaps represent unoccupied positions, the CDR-IMGT lengths (shown between brackets and separated by dots, e.g. [8.8.13]) become crucial information. The IMGT universal numbering is used in 2D graphical representations, designated as IMGT Colliers de Perles [Ruiz, M. and Lefranc, M.-P., Immunogenetics, 53, 857-883 (2002) / Kaas, Q. and Lefranc, M.-P., Current Bioinformatics, 2, 21-30 (2007)], and in 3D structures in IMGT/3Dstructure-DB [Kaas, Q., Ruiz, M. and Lefranc, M.-P., T cell receptor and MHC structural data. Nucl. Acids. Res., 32, D208-D210 (2004)]. The positions of CDRs within a canonical antibody variable domain have been determined by comparison of numerous structures (Al-Lazikani et al., J. Mol. Biol. 273:927-948 (1997); Morea et al., Methods 20:267-279 (2000)). Because the number of residues within a hypervariable region varies in different antibodies, additional residues relative to the canonical positions are conventionally numbered with a, b, c and so forth next to the residue number in the canonical variable domain numbering scheme (Al-Lazikani *et al., supra* (1997)). Such nomenclature is similarly well known to those skilled in the art.

Hypervariable regions may comprise "extended hypervariable regions" as follows: 24-36 or 24-34 (L1), 46-56 or 50-56 (L2) and 89-97 or 89-96 (L3) in the VL and 26-35 or 26-35A (H1), 50-65 or 49-65 (H2) and 93-102, 94-1 02, or 95-102 (H3) in the VH. The variable domain residues are 25 numbered according to Kabat *et al., supra,* for each of these definitions. As used herein, the terms "HVR" and "CDR" are used interchangeably.

As used herein, a "checkpoint inhibitor" refers to a molecule, such as e.g., a small molecule, a soluble receptor, or an antibody, which targets an immune checkpoint and blocks the function of said immune checkpoint. More specifically, a "checkpoint inhibitor" as used herein is a molecule, such as e.g., a small molecule, a soluble receptor, or an antibody, that is capable of inhibiting or otherwise decreasing one or more of the biological activities of an immune checkpoint. An inhibitor of an immune checkpoint protein (e.g., an antagonistic antibody provided herein) can, for example, act by inhibiting or otherwise decreasing the activation and/or cell signalling pathways of the cell expressing said immune checkpoint protein (e.g., a T cell), thereby inhibiting a biological activity of the cell relative to the biological activity in the absence of the antagonist. Example of immune checkpoint inhibitors include small molecule drugs, soluble receptors, and antibodies.

The term "constant region" or "constant domain" refers to a carboxy terminal portion of the light and heavy chain which is not directly involved in binding of the antibody to antigen but exhibits various effector function, such as interaction with the Fc receptor. The terms refer to the portion of an immunoglobulin molecule having a more conserved amino acid sequence relative to the other portion of the immunoglobulin, the variable domain, which contains the antigen binding site. The constant domain contains the CH1, CH2 and CH3 domains of the heavy chain and the CL domain of the light chain.

The term "decreased", as used herein, refers to the level of a biomarker, e.g. progastrin, of a subject at least 1-fold (e.g. 1, 2, 3, 4, 5, 10, 20, 30, 40, 50, 60, 70, 80, 90, 100, 1000, 10,000- fold or more) lower than its reference value. "Decreased", as it refers to the level of a biomarker, e.g. progastrin, of a subject, signifies also at least 5% lower (e.g. 5%, 6%, 7%, 8%, 9%, 10%, 15%, 20%, 25%, 30%, 35%, 40%, 45%, 50%, 55%, 60%, 65%, 70%, 75%, 80%, 85%, 90%), 95%), 99%), or 100%) than the level in the reference sample or with respect to the reference value for said marker.

The term "detecting" as used herein encompasses quantitative or qualitative detection.

The term "detectable probe" or "detectable agent," as used herein, refers to a composition that provides a detectable signal. The term refers to a substance that can be used to ascertain the existence or presence of a desired molecule, such as an antibody provided herein, in a sample or subject. A detectable agent can be a substance that is capable of being visualised or a substance that is otherwise able to be determined and/or measured (e.g., by quantitation). The term includes, without limitation, any fluorophore, chromophore, radiolabel, enzyme, antibody or antibody fragment, and the like, that provide a detectable signal via its activity.

As used herein, "diagnosis" or "identifying a subject having" refers to a process of identifying a disease, condition, or injury from its signs and symptoms. A diagnosis is notably a process of determining if an individual is afflicted with a disease or ailment (e.g., cancer). Cancer is diagnosed for example by detecting either the presence of a marker associated with cancer such as, e.g., progastrin.

The term "diagnostic agent" refers to a substance administered to a subject that aids in the diagnosis of a disease. Such substances can be used to reveal, pinpoint, and/or define the localisation of a disease-causing process. Preferably a diagnostic agent includes a substance that is conjugated to an antibody provided herein, that when administered to a subject or contacted to a sample from a subject, aids in the diagnosis of cancer, tumour formation, or any other cell proliferative disease, disorder or condition.

The term "encode" or grammatical equivalents thereof as it is used in reference to nucleic acid molecule refers to a nucleic acid molecule in its native state or when manipulated by methods well known to those skilled in the art that can be transcribed to produce mRNA, which is then translated into a polypeptide and/or a fragment thereof. The antisense strand is the complement of such a nucleic acid molecule, and the encoding sequence can be deduced therefrom.

An "effective amount" of an agent, e.g., a pharmaceutical formulation, refers to an amount effective, at dosages and for periods of time necessary, to achieve the desired therapeutic or prophylactic result. An effective amount can be administered in one or more administrations, applications or dosages. Such delivery is dependent on a number of variables including the time period for which the individual dosage unit is to be used, the bioavailability of the agent, the route of administration, *etc.* In some instances, effective amount also refers to the amount of an antibody provided herein to achieve a specified result (e.g., inhibition of an immune checkpoint biological activity, such as modulating T cell activation). In some instances, this term refers to the amount of a therapy (e.g., an immune checkpoint inhibitor) which is sufficient to reduce and/or ameliorate the severity and/or duration of a given disease, disorder or condition and/or a symptom related thereto. This term also encompasses an amount necessary for the reduction or amelioration of the advancement or progression of a given disease, disorder or condition, reduction or amelioration of the recurrence, development or onset of a given disease, disorder or condition, and/or to improve or enhance the prophylactic or therapeutic effect(s) of another therapy (e.g., a therapy other than said immune checkpoint inhibitor). In some instances, the effective amount of an antibody is from about 0.1 mg/kg (mg of antibody per kg weight of the subject) to about 100 mg/kg. In some instances, an effective amount of an antibody provided therein is about 0.1 mg/kg, about 0.5 mg/kg, about 1 mg/kg, 3 mg/kg, 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg about 90 mg/kg or about 100 mg/kg (or a range therein).

The term "epitope" as used herein refers to the region of an antigen, such as progastrin polypeptide or progastrin polypeptide fragment, to which an antibody binds. Preferably, an epitope as used herein is a localised region on the surface of an antigen, such as progastrin polypeptide or progastrin polypeptide fragment, that is capable of being bound to one or more antigen binding regions of an antibody, and that has antigenic or immunogenic activity in an animal, such as a mammal (e.g., a human), that is capable of eliciting an immune response. An epitope having immunogenic activity is a portion of a polypeptide that elicits an antibody response in an animal. An epitope having antigenic activity is a portion of a polypeptide to which an antibody binds as determined by any method well known in the art, for example, by an immunoassay. Antigenic epitopes need not necessarily be immunogenic. Epitopes usually consist of chemically active surface groupings of molecules such as amino acids and have specific three-dimensional structural characteristics as well as specific charge characteristics. In certain instances, epitopes may include determinants that are chemically active surface groupings of molecules such as sugar side chains, phosphoryl groups, or sulfonyl groups, and, in certain instances, may have specific three-dimensional structural characteristics, and/or specific charge characteristics. An epitope can be formed by contiguous residues or by non-contiguous residues brought into close proximity by the folding of an antigenic protein. Epitopes formed by contiguous amino acids are typically retained on exposure to denaturing solvents, whereas epitopes formed by non-contiguous amino acids are typically lost under said exposure. Generally, an antigen has several or many different epitopes and reacts with many different antibodies. The determination of the epitope bound by an antibody may be performed by any epitope mapping technique known to a person skilled in the art.

The term "heavy chain" when used in reference to an antibody refers to a polypeptide chain of about 50-70 kDa, wherein the amino-terminal portion includes a variable region of about 120 to 130 or more amino acids and a carboxy-terminal portion that includes a constant region. The constant region can be one of five distinct types, referred to as alpha (α), delta (δ), epsilon (ε), gamma (γ) and mu (µ), based on the amino acid sequence of the heavy chain constant region. The distinct heavy chains differ in size: α, δ and γ contain approximately 450 amino acids, while µ and ε contain approximately 550 amino acids. When combined with a light chain, these distinct types of heavy chains give rise to five well known classes of antibodies, IgA, IgD, IgE, IgG and IgM, respectively, including four subclasses of IgG, namely IgG1, IgG2, IgG3 and IgG4. A heavy chain can be a human heavy chain.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "humanised" antibody refers to a chimeric antibody that contains minimal sequence derived from non-human immunoglobulin. Preferably, a humanised antibody is a human immunoglobulin (recipient antibody) in which residues from a CDR of the recipient are replaced by residues from a CDR of a non-human species (donor antibody) such as mouse, rat, rabbit, or nonhuman primate having the desired specificity, affinity, and/or capacity. In some instances, some of the skeleton segment residues (called FR for framework) can be modified to preserve binding affinity, according to techniques known by a man skilled in the art (Jones et al., Nature, 321:522-525, 1986). In some instances, FR residues of the human immunoglobulin are replaced by corresponding non-human residues. In certain instances, a humanised antibody will comprise substantially all of at least one, and typically two, variable domains, in which all or substantially all of the CDRs correspond to those of a non-human antibody, and all or substantially all of the FRs correspond to those of a human antibody. A humanised antibody optionally may comprise at least a portion of an antibody constant region (Fc), typically that of a human immunoglobulin. A "humanised form" of an antibody, e.g., a non-human antibody, refers to an antibody that has undergone humanisation. The goal of humanisation is a reduction in the immunogenicity of a xenogenic antibody, such as a murine antibody, for introduction into a human, while maintaining the full antigen binding affinity and specificity of the antibody. For further details, see, e.g., Jones et al, Nature 321: 522-525 (1986); Riechmann et al., Nature 332:323-329 (1988); and Presta, Curr. Op. Struct. Biol. 2:593-596 (1992). See also, e.g., Vaswani and Hamilton, Ann. Allergy, Asthma & Immunol. 1 :105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409

As used herein, "identifying" as it refers to a subject that has a condition refers to the process of assessing a subject and determining that the subject has a condition, for example, suffers from cancer.

As used herein, the terms "immune checkpoint" or "immune checkpoint protein" refer to certain proteins made by some types of immune system cells, such as T cells, and some cancer cells. Such proteins regulate T cell function in the immune system. Notably, they help keep immune responses in check and can keep T cells from killing cancer cells. Said immune checkpoint proteins achieve this result by interacting with specific ligands which send a signal into the T cell and essentially switch off or inhibit T cell function. Inhibition of these proteins results in restoration of T cell function and an immune response to the cancer cells. Examples of checkpoint proteins include, but are not limited to CTLA-4, PDL1, PDL2, PD1, B7-H3, B7-H4, BTLA, HVEM, TIM3, GAL9, LAG3, VISTA, KIR, 2B4 (belongs to the CD2 family of molecules and is expressed on all NK, γδ, and memory CD8+ (αB) T cells), CD 160 (also referred to as BY55), CGEN-15049, CHK 1 and CHK2 kinases, IDO1, A2aR and various B-7 family ligands.

The term "increased", as used herein, refers to the level of a biomarker, e.g. progastrin, of a subject at least 1-fold (e.g. 1, 2, 3, 4, 5, 10, 20, 30, 40, 50, 60, 70, 80, 90, 100, 1000, 10,000- fold or more) greater than its reference value. "Increased", as it refers to the level of a biomarker, e.g. progastrin, of a subject, signifies also at least 5% greater (e.g. 5%, 6%, 7%, 8%, 9%, 10%, 15%, 20%, 25%, 30%, 35%, 40%, 45%, 50%, 55%, 60%, 65%, 70%, 75%, 80%, 85%, 90%), 95%), 99%), or 100%) than the level in the reference sample or with respect to the reference value for said marker.

As used herein, an "inhibitor" or "antagonist" refers to a molecule that is capable of inhibiting or otherwise decreasing one or more of the biological activities of a target protein, such as any one of the immune checkpoint proteins described above.

An "isolated" antibody is one which has been separated from a component of its natural environment. In some instances, an antibody is purified to greater than 95% or 99% purity as determined by, for example, electrophoresis (e.g., SDS-PAGE, isoelectric focusing (IEF), capillary electrophoresis) or chromatography (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As intended herein, the "level" of a biomarker, e.g. progastrin, consists of a quantitative value of the said prognosis marker in a sample, e.g. in a sample collected from a cancer-suffering patient. In some instances, the said quantitative value does not consist of an absolute value that is actually measured, but rather consists of a final value resulting from the taking into consideration of a signal to noise ratio occurring with the assay format used, and/or the taking into consideration of calibration reference values that are used to increase reproducibility of the measures of the level of a cancer marker, from assay-to-assay. In some instances, the "level"" of a biomarker, e.g. progastrin, is expressed as arbitrary units, since what is important is that the same kind of arbitrary units are compared (i) from assay-to-assay, or (ii) from one cancer-suffering patient to others, or (iii) from assays performed at distinct time periods for the same patient, or (iv) between the biomarker level measured in a patient's sample and a predetermined reference value (which may also be termed a "cut-off" value herein).

The term "light chain" when used in reference to an antibody refers to a polypeptide chain of about 25 kDa, wherein the amino-terminal portion includes a variable region of about 100 to about 110 or more amino acids and a carboxy-terminal portion that includes a constant region. The approximate length of a light chain is 211 to 217 amino acids. There are two distinct types, referred to as kappa (κ) of lambda (λ) based on the amino acid sequence of the constant domains. Light chain amino acid sequences are well known in the art. A light chain can be a human light chain.

As used herein, "monitoring disease progression" refers to a process of determining the severity or stage of a disease in an individual afflicted with the disease or ailment (e.g., cancer).

As used herein, the term "monoclonal antibody" designates an antibody arising from a nearly homogeneous antibody population, wherein population comprises identical antibodies except for a few possible naturally-occurring mutations which can be found in minimal proportions. A monoclonal antibody arises from the growth of a single cell clone, such as a hybridoma, and is characterised by heavy chains of one class and subclass, and light chains of one type.

As used herein, the "percentage identity" or "% identity" between two sequences of nucleic acids or amino acids refers to the percentage of identical nucleotides or amino acid residues between the two sequences to be compared, obtained after optimal alignment, this percentage being purely statistical and the differences between the two sequences being distributed randomly along their length. The comparison of two nucleic acid or amino acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of methods known by a man skilled in the art.

For the amino acid sequence exhibiting at least 70%, at least 75%, at least 80%, at least 85%, at least 90%, at least 91%, at least 92%, at least 93%, at least 94%, at least 95%, at least 96%, at least 97%, at least 98%, or at least 99% identity with a reference amino acid sequence, preferred examples include those containing the reference sequence, certain modifications, notably a deletion, addition or substitution of at least one amino acid, truncation or extension. In the case of substitution of one or more consecutive or non-consecutive amino acids, substitutions are preferred in which the substituted amino acids are replaced by "equivalent" amino acids. Here, the expression "equivalent amino acids" is meant to indicate any amino acids likely to be substituted for one of the structural amino acids without however modifying the biological activities of the corresponding antibodies and of those specific examples defined below. Equivalent amino acids can be determined either on their structural homology with the amino acids for which they are substituted or on the results of comparative tests of biological activity between the various antibodies likely to be generated.

As used herein, the term "polyclonal antibody" refers to an antibody which was produced among or in the presence of one or more other, non-identical antibodies. In general, polyclonal antibodies are produced from a B-lymphocyte in the presence of several other B-lymphocytes producing non-identical antibodies. Usually, polyclonal antibodies are obtained directly from an immunised animal.

The term "progastrin" as used herein refers to the mammalian progastrin peptide, and particularly human progastrin. For the avoidance of doubt, without any specification, the expression "human progastrin" or "hPG" refers to human PG of sequence SEQ ID No. 1. Human progastrin comprises notably a N-terminus domain and a C-terminus domain which are not present in the biologically active gastrin hormone forms mentioned above. Preferably, the sequence of said N-terminus domain is represented by SEQ ID NO. 2. Preferably, the sequence of said C-terminus domain is represented by SEQ ID NO. 3.

By "progastrin-binding molecule", it is herein referred to any molecule that binds progastrin, but does not bind gastrin-17 (G17), gastrin-34 (G34), glycine-extended gastrin-17 (G17-Gly), or glycine-extended gastrin-34 (G34-Gly) and C-terminal flanking peptide (CTFP). The progastrin-binding molecule disclosed herein may be any progastrin-binding molecule, such as, for instance, an antibody molecule or a receptor molecule. Preferably, the progastrin-binding molecule is an anti-progastrin antibody (an anti-hPG antibody) or an antigen-binding fragment thereof.

As used herein, "prognosis" refers to a process of predicting the probable course and outcome of a disease in an individual afflicted with a disease or ailment (e.g., cancer), or the likelihood of recovery of an individual from a disease (e.g., cancer). "Prognosis" as used herein notably means the likelihood of recovery from a disease or the prediction of the probable development or outcome of a disease. For example, if a sample from a subject is negative for the presence of progastrin, then the "prognosis" for that subject is better than if the sample is positive for progastrin.

The term "reference value", as used herein, refers to the expression level of a biomarker under consideration (e.g. progastrin) in a reference sample. A "reference sample", as used herein, means a sample obtained from subjects, preferably two or more subjects, known to be free of the disease or, alternatively, from the general population. The suitable reference expression levels of biomarker can be determined by measuring the expression levels of said biomarker in several suitable subjects, and such reference levels can be adjusted to specific subject populations. The reference value or reference level can be an absolute value; a relative value; a value that has an upper or a lower limit; a range of values; an average value; a median value, a mean value, or a value as compared to a particular control or baseline value. A reference value can be based on an individual sample value such as, for example, a value obtained from a sample from the subject being tested, but at an earlier point in time. The reference value can be based on a large number of samples, such as from population of subjects of the chronological age matched group, or based on a pool of samples including or excluding the sample to be tested.

As used herein, a "response" refers to an improvement due to treatment. Said improvement can be detected through the observation of clinical symptoms. It will be appreciated that, although not precluded, observing such an improvement does not require that the disorder, condition or symptoms associated therewith be completely eliminated. The types of response a patient can have are a complete response (CR), a partial response (PR), progressive disease (PD), and stable disease (SD).

As used herein, "selecting" refers to the process of determining that an identified subject will receive an agent to treat the occurrence of a condition (e.g., cancer). Selecting can be based on an individual's susceptibility to a particular disease or condition due to, for example, family history, lifestyle, age, ethnicity, or other factors.

A "small molecule drug" is broadly used herein to refer to an organic, inorganic, or organometallic compound typically having a molecular weight of less than about 1000. Small molecule drugs may encompass oligopeptides and other biomolecules having a molecular weight of less than about 1000.

By "soluble receptor", it is herein referred to a peptide or a polypeptide comprising the extracellular domain of a receptor, but not the transmembrane or the cytoplasmic domains thereof.

A "subject" which may be subjected to the methodology described herein may be any of mammalian animals including human, dog, cat, cattle, goat, pig, swine, sheep and monkey. A human subject can be known as a patient. Specifically, "subject" or "subject in need" refers to a mammal that is suffering from cancer or is suspected of suffering from cancer or has been diagnosed with cancer. As used herein, a "cancer-suffering subject" refers to a mammal that is suffering from cancer or has been diagnosed with cancer. A "control subject" refers to a mammal that is not suffering from cancer, and is not suspected of suffering from cancer.

As used herein, "treating" a disease in a subject or "treating" a subject having a disease refers to subjecting the subject to a pharmaceutical treatment, e.g., the administration of a drug, such that the extent of the disease is decreased or prevented. For example, treating results in the reduction of at least one sign or symptom of the disease or condition. Treatment includes (but is not limited to) administration of a composition, such as a pharmaceutical composition, and may be performed either prophylactically, or subsequent to the initiation of a pathologic event. Treatment can require administration of an agent and/ or treatment more than once.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### Methods of diagnosis

Human pre-progastrin, a 101 amino acids peptide (Amino acid sequence reference: AAB19304.1), is the primary translation product of the gastrin gene. Progastrin is formed by cleavage of the first 21 amino acids (the signal peptide) from preprogastrin. The 80 amino-acid chain of progastrin is further processed by cleavage and modifying enzymes to several biologically active gastrin hormone forms: gastrin 34 (G34) and glycine-extended gastrin 34 (G34-Gly), comprising amino acids 38-71 of progastrin, gastrin 17 (G17) and glycine-extended gastrin 17 (G17-Gly), comprising amino acids 55 to 71 of progastrin.

Progastrin (PG) is produced by colorectal tumour cells and is thought to stimulate proliferation of these cells by triggering a signal transduction pathway that blocks the cells' normal differentiation processes, including those processes that lead to cell death. Depletion of the gastrin gene transcript that encodes progastrin induces cell differentiation and programmed cell death in tumour cells in *in vitro* and *in vivo* cancer models, reducing tumour cell proliferation. While not intending to be bound by any theory of operation, through binding of PG, anti-hPG antibodies are thought to block or inhibit its ability to interact with its signalling partner(s). This, in turn, inhibits a signal transduction pathway in colorectal tumour cells that would otherwise lead to proliferation. PG has previously been shown to be a particularly useful tool for diagnosing cancer. See e.g. WO 2011/083 088 for colorectal cancer, WO 2011/083 090 for breast cancer, WO 2011/083 091 for pancreatic cancer, WO 2011/116 954 for colorectal and gastrointestinal cancer, WO 2012/013 609 and WO 2011/083089 for liver pathologies, WO2017114972 for ovarian cancer, WO2017114976 for esophageal cancer, WO2017114975 for gastric cancer, WO2018178364 for lung cancer and WO2018178352 for prostate cancer.

The present application now discloses progastrin as a clinically important negative predictive marker for likelihood of responding to treatment with an immune checkpoint inhibitor. The present inventors have found, surprisingly, that detectable progastrin levels in the fluids before treatment indicate that a patient is less likely to respond to the therapy. Indeed, the inventors have found that this category of patients shows an overall survival which is significantly reduced. In comparison, patients with no detectable progastrin in the fluids before treatment live twice as long.

This represents an important and medically useful discovery. This discovery enables the discrimination of patients, prior to treatment, into a group of patients that is likely to respond to treatment with an immune checkpoint inhibitor and a group of patients that will most probably not respond and will thus require specific and targeted therapeutic treatment. Determining that a patient is likely not to respond to treatment with an immune checkpoint inhibitor may assist physicians in deciding on another therapy which is more likely to be efficacious against the cancer. This diagnosing tool may thus save such patients from expensive treatment with significant side effects whilst ensuring that they receive the most efficacious therapy in their situation.

The present disclosure now provides methods for the *in vitro* identification of cancer patients susceptible to responding to immunotherapy, wherein said method comprises the detection progastrin in a biological sample from a subject. Preferably, the amount of progastrin in said sample is determined, thus allowing quantification of progastrin.

In a first aspect, the present disclosure relates to a method of selection of a cancer patient having an immune-checkpoint-inhibitor responsive phenotype, wherein said method comprises a step of detecting progastrin in a biological sample from a subject. The presence of progastrin in the sample indicates that the patient displays an immune-checkpoint-inhibitor non-responsive phenotype.

Thus, the disclosure relates to an *in vitro* method for selecting a cancer patient having an immune-checkpoint-inhibitor responsive or non-responsive phenotype, said method comprising the steps of:
a) contacting a biological sample from said subject with at least one progastrin-binding molecule, and
b) detecting the binding of said progastrin-binding molecule to progastrin in said sample, wherein said binding indicates the patient displays an immune-checkpoint-inhibitor non-responsive phenotype.

The binding of progastrin-binding molecule may be detected by various assays available to the skilled artisan. Although any suitable means for carrying out the assays as detailed below are included within the disclosure, immunoassays, notably ELISA, can be mentioned in particular.

As used herein, an "immune-checkpoint-inhibitor responsive or non-responsive phenotype" refers to the response state of the subject to the administration of said immune checkpoint inhibitor. A "response state" means that said subject (referred to an immune-checkpoint-inhibitor (non-)responsive phenotype or a (non-)responding subject or a (non-)responsive subject: for the purposes of this application, these terms are essentially synonymous) responds or not to the treatment.

More particularly, a concentration of progastrin of at least 3 pM, at least 5 pM, at least 10 pM, at least 20 pM, at least 30 pM, in said biological sample is indicative of an immune-checkpoint-inhibitor non-responsive phenotype.

In another aspect, the present disclosure relates to an *in vitro* method for the selection of a cancer patient susceptible to responding to treatment with an immune checkpoint inhibitor, wherein said method comprises a step of detecting progastrin in a biological sample from a subject. A cancer patient susceptible to responding to treatment with an immune checkpoint inhibitor is a subject who will display an immune-checkpoint-inhibitor responsive phenotype when administered said immune checkpoint inhibitor. The presence of progastrin in the sample thus indicates that the patient is not susceptible to be responsive to treatment with an immune checkpoint inhibitor.

Thus, the disclosure also relates to an *in vitro* method for selecting a cancer patient susceptible to responding to treatment with an immune checkpoint inhibitor, said method comprising the steps of:
a) contacting a biological sample from said subject with at least one progastrin-binding molecule, and
b) detecting the binding of said progastrin-binding molecule to progastrin in said sample, wherein said binding indicates the patient is not responsive to treatment with an immune checkpoint inhibitor.

Preferably, the method described herein for the *in vitro* selection of a cancer patient susceptible to responding to treatment with an immune checkpoint inhibitor, comprises the steps of:
a) contacting said biological sample from said subject with at least one progastrin-binding molecule,
b) determining the concentration of progastrin in said biological sample, wherein a concentration of progastrin of at least 3 pM in said biological sample is indicative of the absence of responsiveness to treatment with an immune checkpoint inhibitor.

Once the concentration of progastrin present in the sample is determined, the result can be compared with those of control sample(s), which is (are) obtained in a manner similar to the test samples but from individual(s)s known to suffer from a cancer and to be non-responsive to treatment with an immune checkpoint inhibitor. If the concentration of progastrin is significantly more elevated in the test sample, it may be concluded that there is an increased likelihood that the subject from whom it was derived is not responsive to treatment with an immune checkpoint inhibitor. In another instance, the concentration of progastrin present in the sample can be compared with those of control sample(s), which is (are) obtained in a manner similar to the test samples but from individual(s)s known to suffer from a cancer and to be responsive to treatment with an immune checkpoint inhibitor. If the concentration of progastrin is significantly lower in the test sample, it may be concluded that there is an increased likelihood that the subject from whom it was derived is responsive to treatment with an immune checkpoint inhibitor.

Thus, more preferably, the present method comprises the further steps of:
c) determining a reference concentration of progastrin in a reference sample,
d) comparing the concentration of progastrin in said biological sample with said reference concentration of progastrin,
e) determining, from the comparison of step d), whether said patient is responsive or not to treatment with an immune checkpoint inhibitor.

According to another aspect, the present disclosure relates to a method for the in vitro diagnosis of a cancer responsive to treatment with an immune checkpoint inhibitor in a subject, comprising the determination of the concentration of progastrin in a biological sample. More particularly, the biological sample of said subject is contacted with at least one progastrin-binding molecule, wherein said progastrin-binding molecule is an antibody, or an antigen-binding fragment thereof.

Accordingly, disclosure also provides an *in vitro* method for diagnosing a cancer responsive to treatment with an immune checkpoint inhibitor in a subject, said method comprising the steps of:
a) contacting a biological sample from said subject with at least one progastrin-binding molecule, and
b) detecting the binding of said progastrin-binding molecule to progastrin in said sample, wherein said binding indicates the cancer is not a cancer responsive to treatment with an immune checkpoint inhibitor.

Preferably, the present disclosure relates to a method for the *in vitro* diagnosis of a cancer responsive to treatment with an immune checkpoint inhibitor in a subject, comprising the steps of:
a) contacting said biological sample from said subject with at least one progastrin-binding molecule,
b) determining concentration of progastrin in said biological sample, wherein a concentration of progastrin of at least 3 pM in said biological sample is indicative of the presence of a cancer not responsive to treatment with an immune checkpoint inhibitor.

More particularly, a concentration of progastrin of at least 3 pM, at least 5 pM, at least 10 pM, at least 20 pM, at least 30 pM, in said biological sample is indicative of the presence of a cancer which is not responsive to treatment with an immune checkpoint inhibitor in said subject.

More preferably, diagnosing a cancer responsive to treatment with an immune checkpoint inhibitor in a subject involves comparing the concentration of progastrin measured in said biological sample of the subject to the concentration of progastrin in a reference sample.

Accordingly, the present method comprises the further steps of:
c) determining a reference concentration of progastrin in a reference sample,
d) comparing the concentration of progastrin in said biological sample with said reference level or concentration of progastrin,
e) diagnosing, from the comparison of step d), whether said cancer is responsive or not to treatment with an immune checkpoint inhibitor.

According to another aspect, the disclosure relates to an *in vitro* method for diagnosing a metastasised cancer responsive to treatment with an immune checkpoint inhibitor in a subject, said method comprising the steps of:
a) contacting biological sample from said subject with at least one progastrin-binding molecule, and
b) detecting the binding of said progastrin-binding molecule to progastrin in said sample, wherein said binding indicates a metastasised cancer not responsive to treatment with an immune checkpoint inhibitor.

Preferably, the present disclosure relates to a method for the *in vitro* diagnosis of a metastasised cancer responsive to treatment with an immune checkpoint inhibitor in a subject, from a biological sample of said subject, comprising the steps of:
a) contacting said biological sample with at least one progastrin-binding molecule,
b) determining by a biochemical assay the level or concentration of progastrin in said biological sample, wherein a concentration of progastrin of at least 3 pM in said biological sample is indicative of the presence of a metastasised cancer not responsive to treatment with an immune checkpoint inhibitor in said subject.

More particularly, a concentration of progastrin of at least 3 pM, at least 5 pM, at least 10 pM, at least 20 pM, at least 30 pM, in said biological sample is indicative of the presence of a metastasised cancer which is not responsive to treatment with an immune checkpoint inhibitor in said subject.

More preferably, the present method comprises the further steps of:
c) determining a reference concentration of progastrin in a reference sample,
d) comparing the concentration of progastrin in said biological sample with said reference concentration of progastrin,
e) diagnosing, from the comparison of step d), whether said cancer is responsive or not to treatment with an immune checkpoint inhibitor.

According to another aspect, the present disclosure relates to a method for the in vitro prognosis of a cancer treatment with an immune checkpoint inhibitor in a subject, comprising the determination of the concentration of progastrin in a biological sample. More particularly, the biological sample of said subject is contacted with at least one progastrin-binding molecule, wherein said progastrin-binding molecule is an antibody, or an antigen-binding fragment thereof.

Accordingly, the disclosure also provides an *in vitro* method for prognosing a cancer treatment with an immune checkpoint inhibitor in a subject, said method comprising the steps of:
a) contacting a biological sample from said subject with at least one progastrin-binding molecule, and
b) detecting the binding of said progastrin-binding molecule to progastrin in said sample, wherein said binding indicates the prognosis is negative.

Preferably, the present disclosure relates to a method for the *in vitro* prognosis of a cancer treatment with an immune checkpoint inhibitor in a subject, comprising the steps of:
a) contacting said biological sample from said subject with at least one progastrin-binding molecule,
b) determining concentration of progastrin in said biological sample, wherein a concentration of progastrin of at least 10 pM in said biological sample is indicative of the negative prognosis.

More particularly, a concentration of progastrin of at least 3 pM, at least 5 pM, at least 10 pM, at least 20 pM, at least 30 pM, in said biological sample is indicative of a negative prognosis.

More preferably, prognosing a cancer treatment with an immune checkpoint inhibitor in a subject involves comparing the concentration of progastrin measured in said biological sample of the subject to the concentration of progastrin in a reference sample.

Accordingly, the method of the disclosure comprises the further steps of:
c) determining a reference concentration of progastrin in a reference sample,
d) comparing the concentration of progastrin in said biological sample with said reference level or concentration of progastrin,
e) prognosing, from the comparison of step d), said cancer treatment with an immune checkpoint inhibitor.

### Anti-hPG antibodies

The PG-binding molecules for use in the present methods are molecules that bind to progastrin, including a PG polypeptide, a PG polypeptide fragment, or a PG epitope, but does not bind gastrin-17 (G17), gastrin-34 (G34), glycine-extended gastrin-17 (G17-Gly), or glycine-extended gastrin-34 (G34-Gly) and C-terminal flanking peptide (CTFP). Preferably, the PG-binding molecules bind human progastrin, i.e., the polypeptide of amino acid sequence represented by SEQ ID NO. 1.

The PG-binding-molecule is for example an antibody binding to PG (an anti-PG antibody) or an antigen-binding fragment thereof. Preferably, said an anti-progastrin antibody binds to hPG (an anti-hPG antibody).

Anti-human progastrin (anti-hPG) monoclonal antibodies and their use for diagnosis or therapy have been described in the following documents: WO 2011/083 088 for colorectal cancer, WO 2011/083 090 for breast cancer, WO 2011/083 091 for pancreatic cancer, WO 2011/116 954 for colorectal and gastrointestinal cancer, WO 2012/013 609 and WO 2011/083089 for liver pathologies, WO 2017/114975 for gastric cancer, and WO 2017/114973.

In particular, said progastrin-binding antibody, or an antigen-binding fragment thereof, is selected from the group consisting of: polyclonal antibodies, monoclonal antibodies, chimeric antibodies, single chain antibodies, camelised antibodies, IgA1 antibodies, IgA2 antibodies, IgD antibodies, IgE antibodies, IgG1 antibodies, IgG2 antibodies, IgG3 antibodies, IgG4 antibodies and IgM antibodies.

More specifically, the present anti-PG antibody recognises an epitope of progastrin wherein said epitope includes an amino acid sequence corresponding to an amino acid sequence of the N-terminal part of progastrin, wherein said amino acid sequence may include residues 10 to 14 of hPG, residues 9 to 14 of hPG, residues 4 to 10 of hPG, residues 2 to 10 of hPG or residues 2 to 14 of hPG, wherein the amino acid sequence of hPG is SEQ ID N° 1.

More specifically, the anti-PG antibody recognises an epitope of progastrin wherein said epitope includes an amino acid sequence corresponding to an amino acid sequence of the C-terminal part of progastrin, wherein said amino acid sequence may include residues 71 to 74 of hPG, residues 69 to 73 of hPG, residues 71 to 80 of hPG (SEQ ID N° 40), residues 76 to 80 of hPG, or residues 67 to 74 of hPG, wherein the amino acid sequence of hPG is SEQ ID N°1.

More particularly, the anti-PG antibody has an affinity for progastrin of at least 5000 nM, at least 500 nM, 100 nM, 80 nM, 60 nM, 50 nM, 40 nM, 30 nM, 20 nM, 10 nM, 7 nM, 5 nM, 4 nM, 3 nM, 2 nM, 1 nM, 0.5 nM, 0.1nM, 50 pM, 10 pM, 5 pM, 1 pM, or at least 0.1 pM, as determined by a method such as those described herein.

In another particular instance, the antibody binding to progastrin has been obtained by an immunisation method known by a person skilled in the art, wherein using as an immunogen a peptide which amino acid sequence comprises the totality or a part of the amino-acid sequence of progastrin. More particularly, said immunogen comprises a peptide chosen among:
- a peptide which amino acid sequence comprises, or consists of, the amino acid sequence of full length progastrin, and particularly full length human progastrin of SEQ ID N°1,
- a peptide which amino acid sequence corresponds to a part of the amino acid sequence of progastrin, and particularly full length human progastrin of SEQ ID *N°1,*
- a peptide which amino acid sequence corresponds to a part or to the whole amino acid sequence of the N-terminal part of progastrin, and in particular peptides comprising, or consisting of, the amino acid sequence: SWKPRSQQPDAPLG (SEQ ID N°2), and
- a peptide which amino acid sequence corresponds to a part or to the whole amino acid sequence of the C-terminal part of progastrin, and in particular peptides comprising, or consisting of, the amino acid sequence: QGPWLEEEEEAYGWMDFGRRSAEDEN (SEQ ID N°3)*,*
- a peptide which amino acid sequence corresponds to a part of the amino acid sequence of the C-terminal part of progastrin, and in particular peptides comprising the amino acid sequence FGRRSAEDEN (SEQ ID N°40) corresponding to amino acids 71-80 of progastrin.

The skilled person will realise that such immunisation may be used to generate either polyclonal or monoclonal antibodies, as desired. Methods for obtaining each of these types of antibodies are well known in the art. The skilled person will thus easily select and implement a method for generating polyclonal and/or monoclonal antibodies against any given antigen.

Examples of monoclonal antibodies which were generated by using an immunogen comprising the amino-acid sequence "SWKPRSQQPDAPLG", corresponding to the amino acid sequence 1-14 of human progastrin (N-terminal extremity) include, but are not restricted to, monoclonal antibodies designated as: mAb3, mAb4, mAb16, and mAb19 and mAb20, as described in the following Table 1 to Table 4. Other monoclonal antibodies have been described, although it is not clear whether these antibodies actually bind progastrin (WO 2006/032980). Experimental results of epitope mapping show that mAb3, mAb4, mAb16, and mAb19 and mAb20 do specifically bind an epitope within said hPG N-terminal amino acid sequence (SEQ ID NO. 2). Polyclonal antibodies recognising specifically an epitope within the N-terminus of progastrin represented by SEQ ID NO. 2, have been described in the art (see e.g., WO 2011/083088).

**Table 1**

| Hybridoma deposit | mAb | Amino acid sequences | | SEQ ID N° |
|---|---|---|---|---|
| 6B5B11C10 | mAb3 | VH CDR 1 | GYIFTSYW | SEQ ID N°4 |
| | | VH CDR 2 | FYPGNSDS | SEQ ID N°5 |
| | | VH CDR 3 | TRRDSPQY | SEQ ID N°6 |
| | | VL CDR 1 | QSIVHSNGNTY | SEQ ID N°7 |
| | | VL CDR 2 | KVS | SEQ ID N°8 |
| | | VL CDR 3 | FQGSHVPFT | SEQ ID N°9 |
| | | mVH 3 | EVQLQQSGTVLARPGASVKMSCK ASGYIFTSYWVHWVKQRPGQGLE WIGGFYPGNSDSRYNQKFKGKAT LTAVTSASTAYMDLSSLTNEDSAV YFCTRRDSPQYWGQGTTLTVSS | SEQ ID N° 41 |
| | | mVL 3 | DVLMTQTPLSLPVSLGDQASISCR SSQSIVHSNGNTYLEWYLQKPGQS PKLLIYKVSNRFSGVPDRFSGSGS GTDFTLKISRLEAEDLGVYYCFQG SHVPFTFGGGTKLEIK | SEQ ID N° 42 |
| | | huVH 3 | QVQLVQSGAEVKKPGASVKVSCK ASGYIFTSYWVHWVRQAPGQRLE WMGGFYPGNSDSRYSQKFQGRV TITRDTSASTAYMELSSLRSEDTAV YYCTRRDSPQYWGQGTLVTVSS | SEQ ID N° 53 |
| | | huVL 3 | DVVMTQSPLSLPVTLGQPASISCR SSQSIVHSNGNTYLEWFQQRPGQ SPRRLIYKVSNRFSGVPDRFSGSGS GTDFTLKISRVEAEDVGVYYCFQG SHVPFTFGGGTKVEIK | SEQ ID N° 54 |

**Table 2**

| Hybridoma deposit | mAb | Amino acid sequences | | SEQ ID N° |
|---|---|---|---|---|
| 20D2C3G2 | mAb4 | VH CDR 1 | GYTFSSW | SEQ ID N°10 |
| | | VH CDR 2 | FLPGSGST | SEQ ID N°11 |
| | | VH CDR 3 | ATDGNYDWFAY | SEQ ID N°12 |
| | | VL CDR 1 | QSLVHSSGVTY | SEQ ID N°13 |
| | | VL CDR 2 | KVS | SEQ ID N°14 |
| | | VL CDR 3 | SQSTHVPPT | SEQ ID N°15 |
| | | mVH 4 | QVQLQQSGAELMKPGASVKISCK ATGYTFSSSWIEWLKQRPGHGLE WIGEFLPGSGSTDYNEKFKGKATF TADTSSDTAYMLLSSLTSEDSAVY YCATDGNYDWFAYWGQGTLVTV SA | SEQ ID N° 43 |
| | | mVL 4 | DLVMTQTPLSLPVSLGDQASISCR SSQSLVHSSGVTYLHWYLQKPGQ SPKLLIYKVSNRFSGVPDRFSGSGS GTDFTLKISRVEAEDLGVYFCSQS THVPPTFGSGTKLEIK | SEQ ID N° 44 |
| | | huVH 4 | QVQLVQSGAEVKKPGASVKVSCK ASGYTFSSSWMHWVRQAPGQGL EWMGIFLPGSGSTDYAQKFQGRV TMTRDTSTSTVYMELSSLRSEDTA VYYCATDGNYDWFAYWGQGTLV TVSS | SEQ ID N° 55 |
| | | huVL 4 | DIVMTQTPLSLSVTPGQPASISCKS SQSLVHSSGVTYLYWYLQKPGQS PQLLIYKVSNRFSGVPDRFSGSGS | SEQ ID N° 56 |
| | | | GTDFTLKISRVEAEDVGVYYCSQS THVPPTFGQGTKLEIK | |

**Table 3**

| Hybridoma deposit | mAb | Amino acid sequences | | SEQ ID N° |
|---|---|---|---|---|
| 1E9D9B6 | mAb16 | VH CDR 1 | GYTFTSYY | SEQ ID N°16 |
| | | VH CDR 2 | INPSNGGT | SEQ ID N°17 |
| | | VH CDR 3 | TRGGYYPFDY | SEQ ID N°18 |
| | | VL CDR 1 | QSLLDSDGKTY | SEQ ID N°19 |
| | | VL CDR 2 | LVS | SEQ ID N°20 |
| | | VL CDR 3 | WQGTHSPYT | SEQ ID N°21 |
| | | mVH 16 | QVQLQQSGAELVKPGASVKLSCK ASGYTFTSYYMYWVKQRPGQGLE WIGEINPSNGGTNFNEKFKSKATL TVDKSSSTAYMQLSSLTSEDSAVY YCTRGGYYPFDYWGQGTTLTVSS | SEQ ID N° 45 |
| | | mVL 16 | DVVMTQTPLTLSVTIGRPASISCKS SQSLLDSDGKTYLYWLLQRPGQS PKRLIYLVSELDSGVPDRITGSGSG TDFTLKISRVEAEDLGVYYCWQG THSPYTFGGGTKLEIK | SEQ ID N° 46 |
| | | huVH 16a | QVQLVQSGAEVKKPGASVKVSCK ASGYTFTSYYMYWVRQAPGQGLE WMGIINPSNGGTSYAQKFQGRVT MTRDTSTSTVYMELSSLRSEDTAV | SEQ ID N° 57 |
| | | | YYCTRGGYYPFDYWGQGTTVTV SS | |
| | | huVH 16b | QVQLVQSGAEVKKPGASVKVSCK ASGYTFTSYYMHWVRQAPGQGL EWMGIINPSNGGTSYAQKFQGRV TMTRDTSTSTVYMELSSLRSEDTA VYYCTRGGYYPFDYWGQGTTVT VSS | SEQ ID N° 58 |
| | | huVH 16c | QVQLVQSGAEVKKPGASVKVSCK ASGYTFTSYYMYWVRQAPGQGLE WMGEINPSNGGTNYAQKFQGRV TMTRDTSTSTVYMELSSLRSEDTA VYYCTRGGYYPFDYWGQGTTVT VSS | SEQ ID N° 59 |
| | | huVL 16a | DVVMTQSPLSLPVTLGQPASISCR SSQSLLDSDGKTYLYWFQQRPGQ SPRRLIYLVSNRDSGVPDRFSGSGS GTDFTLKISRVEAEDVGVYYCWQ GTHSPYTFGQGTKLEIK | SEQ ID N° 60 |
| | | huVL 16b | DVVMTQSPLSLPVTLGQPASISCR SSQSLLDSDGKTYLNWFQQRPGQ SPRRLIYLVSNRDSGVPDRFSGSGS GTDFTLKISRVEAEDVGVYYCWQ GTHSPYTFGQGTKLEIK | SEQ ID N° 61 |
| | | huVL 16c | DVVMTQSPLSLPVTLGQPASISCR SSQSLLDSDGKTYLYWFQQRPGQ SPRRLIYLVSERDSGVPDRFSGSGS GTDFTLKISRVEAEDVGVYYCWQ GTHSPYTFGQGTKLEIK | SEQ ID N° 62 |

**Table 4**

| Hybridoma deposit | mAb | Amino acid sequences | | SEQ ID N° |
|---|---|---|---|---|
| 1B3B4F11 | mAb19 | VH CDR 1 | GYSITSDYA | SEQ ID N°22 |
| | | VH CDR 2 | ISFSGYT | SEQ ID N°23 |
| | | VH CDR 3 | AREVNYGDSYHFDY | SEQ ID N°24 |
| | | VL CDR 1 | SQHRTYT | SEQ ID N°25 |
| | | VL CDR 2 | VKKDGSH | SEQ ID N°26 |
| | | VL CDR 3 | GVGDAIKGQSVFV | SEQ ID N°27 |
| | | mVH 19 | DVQLQESGPGLVKPSQSLSLTCTVT GYSITSDYAWNWIRQFPGNKLEWM GYISFSGYTSYNPSLKSRISVTRDTS RNQFFLQLTSVTTEDTATYYCARE VNYGDSYHFDYWGQGTIVTVSS | SEQ ID N° 47 |
| | | mVL 19 | QLALTQSSSASFSLGASAKLTCTLSS QHRTYTIEWYQQQSLKPPKYVMEV KKDGSHSTGHGIPDRFSGSSSGADR YLSISNIQPEDEAIYICGVGDAIKGQS VFVFGGGTKVTVL | SEQ ID N° 48 |
| | | huVH 19a | QVQLQESGPGLVKPSQTLSLTCTVS GYSITSDYAWNWIRQHPGKGLEWI GYISFSGYTYYNPSLKSRVTISVDTS KNQFSLKLSSVTAADTAVYYCAREV NYGDSYHFDYWGQGTLVTVSS | SEQ ID N° 63 |
| | | huVH 19b | QVQLQESGPGLVKPSQTLSLTCTVS GYSITSDYAWSWIRQHPGKGLEWI GYISFSGYTYYNPSLKSRVTISVDTS | SEQ ID N° 64 |
| | | | KNQFSLKLSSVTAADTAVYYCAREV NYGDSYHFDYWGQGTLVTVSS | |
| | | huVH 19c | QVQLQESGPGLVKPSQTLSLTCTVS GYSITSDYAWNWIRQHPGKGLEWI GYISFSGYTSYNPSLKSRVTISVDTS KNQFSLKLSSVTAADTAVYYCAREV NYGDSYHFDYWGQGTLVTVSS | SEQ ID N° 65 |
| | | huVL 19a | QLVLTQSPSASASLGASVKLTCTLSS QHRTYTIEWHQQQPEKGPRYLMK VKKDGSHSKGDGIPDRFSGSSSGAE RYLTISSLQSEDEADYYCGVGDAIK GQSVFVFGGGTKVEIK | SEQ ID N° 66 |
| | | huVL 19b | QLVLTQSPSASASLGASVKLTCTLSS QHRTYTIAWHQQQPEKGPRYLMK VKKDGSHSKGDGIPDRFSGSSSGAE RYLTISSLQSEDEADYYCGVGDAIK GQSVFVFGGGTKVEIK | SEQ ID N° 67 |
| | | huVL 19c | QLVLTQSPSASASLGASVKLTCTLSS QHRTYTIEWHQQQPEKGPRYLME VKKDGSHSKGDGIPDRFSGSSSGAE RYLTISSLQSEDEADYYCGVGDAIK GQSVFVFGGGTKVEIK | SEQ ID N° 68 |

Examples of monoclonal antibodies that can be generated by using an immunogen comprising the amino-acid sequence "QGPWLEEEEEAYGWMDFGRRSAEDEN", (C-terminal part of progastrin) corresponding to the amino acid sequence 55-80 of human progastrin include, but are not restricted to antibodies designated as: mAb8 and mAb13 in the following Table 5 and 6. Another example of a monoclonal antibody that can thus be generated by is the antibody Mab14, produced by hybridoma 2H9F4B7, described in WO 2011/083088. Hybridoma 2H9F4B7 was deposited under the Budapest Treaty at the CNCM, Institut Pasteur, 25-28 rue du Docteur Roux, 75724 Paris CEDEX 15, France, on 27 December 2016, under reference I-5158. Experimental results of epitope mapping show that these antibodies do specifically bind an epitope within said hPG C-terminal amino acid sequence (SEQ ID NO. 3).

**Table 5**

| Hybridoma deposit | mAb | Amino acid sequences | | SEQ ID N° |
|---|---|---|---|---|
| 1C10D3B9 | mAb8 | VH CDR 1 | GFTFTTYA | SEQ ID N°28 |
| | | VH CDR 2 | ISSGGTYT | SEQ ID N°29 |
| | | VH CDR 3 | ATQGNYSLDF | SEQ ID N°30 |
| | | VL CDR 1 | KSLRHTKGITF | SEQ ID N°31 |
| | | VL CDR 2 | QMS | SEQ ID N°32 |
| | | VL CDR 3 | AQNLELPLT | SEQ ID N°33 |
| | | mVH 8 | EVQLVESGGGLVKPGGSLRLSC AASGFTFTTYAMSWVRQAPGK GLEWVATISSGGTYTYYADSVK GRFTISRDNAKNSLYLQMNSLRA EDTAVYYCATQGNYSLDFWGQ GTTVTVSS | SEQ ID N° 49 |
| | | mVL 8 | DIVMTQSPLSLPVTPGEPASISCR SSKSLRHTKGITFLYWYLQKPGQ SPQLLIYQMSNLASGVPDRFSSS GSGTDFTLKISRVEAEDVGVYYC AQNLELPLTFGGGTKVEIK | SEQ ID N° 50 |
| | | VH hZ8CV1 | EVQLVESGGGLVKPGGSLRLSC AASGFTFTTYAMSWVRQAPGK GLEWVSSISSGGTYTYYADSVKG RFTISRDNAKNSLYLQMNSLRAE | SEQ ID N° 69 |
| | | | DTAVYYCATQGNYSLDFWGQG TTVTVSS | |
| | | VL hZ8CV1 | DIVMTQSPLSLPVTPGEPASISCR SSKSLRHTKGITFLYWYLQKPGQ SPQLLIYQMSNRASGVPDRFSGS GSGTDFTLKISRVEAEDVGVYYC AQNLELPLTFGGGTKVEIK | SEQ ID N° 70 |
| | | VH hZ8CV2 | EVQLVESGGGLVKPGGSLRLSC AASGFTFTTYAMSWVRQAPGK GLEWVATISSGGTYTYYADSVK GRFTISRDNAKNSLYLQMNSLRA EDTAVYYCATQGNYSLDFWGQ GTTVTVSS | SEQ ID N° 71 |
| | | VL hZ8CV2 | DIVMTQSPLSLPVTPGEPASISCR SSKSLRHTKGITFLYWYLQKPGQ SPQLLIYQMSNLASGVPDRFSSS GSGTDFTLKISRVEAEDVGVYYC AQNLELPLTFGGGTKVEIK | SEQ ID N° 72 |
| | | CH hZ8CV2 | EVQLVESGGGLVKPGGSLRLSC AASGFTFTTYAMSWVRQAPGK GLEWVATISSGGTYTYYADSVK GRFTISRDNAKNSLYLQMNSLRA EDTAVYYCATQGNYSLDFWGQ GTTVTVSSASTKGPSVFPLAPSS KSTSGGTAALGCLVKDYFPEPV TVSWNSGALTSGVHTFPAVLQS SGLYSLSSVVTVPSSSLGTQTYIC NVNHKPSNTKVDKRVEPKSCDK THTCPPCPAPELLGGPSVFLFPP KPKDTLMISRTPEVTCVVVDVSH EDPEVKFNWYVDGVEVHNAKT KPREEQYNSTYRVVSVLTVLHQ | SEQ ID N° 73 |
| | | | DWLNGKEYKCKVSNKALPAPIEK TISKAKGQPREPQVYTLPPSREE MTKNQVSLTCLVKGFYPSDIAVE WESNGQPENNYKTTPPVLDSDG SFFLYSKLTVDKSRWQQGNVFS CSVMHEALHNHYTQKSLSLSPG K | |
| | | CL hZ8CV2 | DIVMTQSPLSLPVTPGEPASISCR SSKSLRHTKGITFLYWYLQKPGQ SPQLLIYQMSNLASGVPDRFSSS GSGTDFTLKISRVEAEDVGVYYC AQNLELPLTFGGGTKVEIKRTVA APSVFIFPPSDEQLKSGTASVVCL LNNFYPREAKVQWKVDNALQSG NSQESVTEQDSKDSTYSLSSTLT LSKADYEKHKVYACEVTHQGLS SPVTKSFNRGEC | SEQ ID N° 74 |

**Table 6**

| Hybridoma deposit | mAb | Amino acid sequences | | SEQ ID N° |
|---|---|---|---|---|
| 2C6C3C7 | mAb13 | VH CDR 1 | GFIFSSYG | SEQ ID N°34 |
| | | VH CDR 2 | INTFGDRT | SEQ ID N°35 |
| | | VH CDR 3 | ARGTGTY | SEQ ID N°36 |
| | | VL CDR 1 | QSLLDSDGKTY | SEQ ID N°37 |
| | | VL CDR 2 | LVS | SEQ ID N°38 |
| | | VL CDR 3 | WQGTHFPQT | SEQ ID N°39 |
| | | mVH 13 | EVQLVESGGGLVQPGGSLKLSC AASGFIFSSYGMSWVRQSPDRRL ELVASINTFGDRTYYPDSVKGRF TISRDNAKNTLYLQMTSLKSEDT AIYYCARGTGTYWGQGTTLTVS S | SEQ ID N° 51 |
| | | mVL 13 | DVVLTQTPLTLSVTIGQPASISCK SSQSLLDSDGKTYLNWLLQRPG QSPKRLIYLVSKLDSGVPDRFTG SGSGTDFTLKISRVEAEDLGVYY CWQGTHFPQTFGGGTKLEIK | SEQ ID N° 52 |
| | | huVH 13a | EVQLVESGGGLVQPGGSLRLSC AASGFIFSSYGMSWVRQAPGKG LEWVANINTFGDRTYYVDSVKG RFTISRDNAKNSLYLQMNSLRAE DTAVYYCARGTGTYWGQGTLV TVSS | SEQ ID N° 75 |
| | | huVH 13b | EVQLVESGGGLVQPGGSLRLSC AASGFIFSSYGMSWVRQAPGKG LEWVASINTFGDRTYYVDSVKG RFTISRDNAKNSLYLQMNSLRAE DTAVYYCARGTGTYWGQGTLV TVSS | SEQ ID N° 76 |
| | | huVL 13a | DVVMTQSPLSLPVTLGQPASISC RSSQSLLDSDGKTYLNWFQQRP GQSPRRLIYLVSNRDSGVPDRFS GSGSGTDFTLKISRVEAEDVGVY YCWQGTHFPQTFGGGTKVEIK | SEQ ID N° 77 |
| | | huVL 13b | DVVMTQSPLSLPVTLGQPASISC RSSQSLLDSDGKTYLNWFQQRP GQSPRRLIYLVSKRDSGVPDRFS | SEQ ID N° 78 |
| | | | GSGSGTDFTLKISRVEAEDVGVY YCWQGTHFPQTFGGGTKVEIK | |

Other examples include anti-hPG monoclonal and/or polyclonal antibodies generated by using an immunogen comprising an amino acid sequence of SEQ ID N° 40.

More particularly, said biological sample is contacted with an anti-hPG antibody or antigen-binding fragment thereof, wherein said anti-hPG antibody is chosen among N-terminal anti-hPG antibodies and C-terminal anti-hPG antibodies.

The terms "N-terminal anti-hPG antibodies" and "C-terminal anti-hPG antibodies" designate antibodies binding to an epitope comprising amino acids located in the N-terminal part of hPG or to an epitope comprising amino acids located in the C-terminal part of hPG, respectively. Preferably, the term "N-terminal anti-hPG antibodies" refers to antibodies binding to an epitope located in a domain of progastrin whose sequence is represented by SEQ ID NO. 2. Preferably, the term "C-terminal anti-hPG antibodies" refers to antibodies binding to an epitope located in a domain of progastrin whose sequence is represented by SEQ ID NO. 3.

In particular, said antibody is a monoclonal antibody selected in the group consisting of:
- A monoclonal antibody comprising a heavy chain comprising at least one, preferentially at least two, preferentially three, of CDR-H1, CDR-H2 and CDR-H3 of amino acid sequences SEQ ID N°4, 5 and 6, respectively, or sequences with at least 80%, preferably 85%, 90%, 95% and 98% identity after optimal alignment with sequences SEQ ID N°4, 5 and 6, respectively, and a light chain comprising at least one, preferentially at least two, preferentially three, of CDR-L1, CDR-L2 and CDR-L3 of amino acid sequences SEQ ID N°7, 8 and 9, respectively, or sequences with at least 80%, preferably 85%, 90%, 95% and 98% identity after optimal alignment with sequences SEQ ID N°7, 8 and 9, respectively,
- A monoclonal antibody comprising a heavy chain comprising at least one, preferentially at least two, preferentially three, of CDR-H1, CDR-H2 and CDR-H3 of amino acid sequences SEQ ID N°10, 11 and 12, respectively, or sequences with at least 80%, preferably 85%, 90%, 95% and 98% identity after optimal alignment with sequences SEQ ID N°10, 11 and 12, respectively, and a light chain comprising at least one, preferentially at least two, preferentially three, of CDR-L1, CDR-L2 and CDR-L3 of amino acid sequences SEQ ID N° 13, 14 and 15, respectively, or sequences with at least 80%, preferably 85%, 90%, 95% and 98% identity after optimal alignment with sequences SEQ ID N° 13, 14 and 15, respectively,
- A monoclonal antibody comprising a heavy chain comprising at least one, preferentially at least two, preferentially three, of CDR-H1, CDR-H2 and CDR-H3 of amino acid sequences SEQ ID N°16, 17 and 18, respectively, or sequences with at least 80%, preferably 85%, 90%, 95% and 98% identity after optimal alignment with sequences SEQ ID N°16, 17 and 18, respectively, and a light chain comprising at least one, preferentially at least two, preferentially three, of CDR-L1, CDR-L2 and CDR-L3 of amino acid sequences SEQ ID N° 19, 20 and 21, respectively, or sequences with at least 80%, preferably 85%, 90%, 95% and 98% identity after optimal alignment with sequences SEQ ID N° 19, 20 and 21, respectively,
- A monoclonal antibody comprising a heavy chain comprising at least one, preferentially at least two, preferentially three, of CDR-H1, CDR-H2 and CDR-H3 of amino acid sequences SEQ ID N°22, 23 and 24, respectively, or sequences with at least 80%, preferably 85%, 90%, 95% and 98% identity after optimal alignment with sequences SEQ ID N°22, 23 and 24, respectively, and a light chain comprising at least one, preferentially at least two, preferentially three, of CDR-L1, CDR-L2 and CDR-L3 of amino acid sequences SEQ ID N° 25, 26 and 27, respectively, or sequences with at least 80%, preferably 85%, 90%, 95% and 98% identity after optimal alignment with sequences SEQ ID N°25, 26 and 27, respectively,
- A monoclonal antibody comprising a heavy chain comprising at least one, preferentially at least two, preferentially at least three, of CDR-H1, CDR-H2 and CDR-H3 of amino acid sequences SEQ ID N°28, 29 and 30, respectively, or sequences with at least 80%, preferably 85%, 90%, 95% and 98% identity after optimal alignment with sequences SEQ ID N°28, 29 and 30, respectively, and a light chain comprising at least one, preferentially at least two, preferentially three, of CDR-L1, CDR-L2 and CDR-L3 of amino acid sequences SEQ ID N°31, 32 and 33, respectively, or sequences with at least 80%, preferably 85%, 90%, 95% and 98% identity after optimal alignment with sequences SEQ ID N°31, 32 and 33, respectively, and

- A monoclonal antibody comprising a heavy chain comprising at least one, preferentially at least two, preferentially three, of CDR-H1, CDR-H2 and CDR-H3 of amino acid sequences SEQ ID N°34, 35 and 36, respectively, or sequences with at least 80%, preferably 85%, 90%, 95% and 98% identity after optimal alignment with sequences SEQ ID N°34, 35 and 36, respectively, and a light chain comprising at least one, preferentially at least two, preferentially three, of CDR-L1, CDR-L2 and CDR-L3 of amino acid sequences SEQ ID N° 37, 38 and 39, respectively, or sequences with at least 80%, preferably 85%, 90%, 95% and 98% identity after optimal alignment with sequences SEQ ID N°37, 38 and 39, respectively.

Specifically, the antibody is a monoclonal antibody produced by the hybridoma deposited at the CNCM, Institut Pasteur, 25-28 rue du Docteur Roux, 75724 Paris CEDEX 15, France, on 27 December 2016, under reference I-5158.

More particularly, said antibody is a monoclonal antibody selected in the group consisting of:
- A monoclonal antibody comprising a heavy chain of amino acid sequence SEQ ID N°41 and a light chain of amino acid sequence SEQ ID N°42;
- A monoclonal antibody comprising a heavy chain of amino acid sequence SEQ ID N°43 and a light chain of amino acid sequence SEQ ID N°44;
- A monoclonal antibody comprising a heavy chain of amino acid sequence SEQ ID N° 45 and a light chain of amino acid sequence SEQ ID N° 46;
- A monoclonal antibody comprising a heavy chain of amino acid sequence SEQ ID N°47 and a light chain of amino acid sequence SEQ ID N° 48;
- A monoclonal antibody comprising a heavy chain of amino acid sequence SEQ ID N°49 and a light chain of amino acid sequence SEQ ID N°50; and
- A monoclonal antibody comprising a heavy chain of amino acid sequence SEQ ID N°51 and a light chain of amino acid sequence SEQ ID N°52.

In another particular instance, the antibody used in the method disclosed herein is a humanised antibody. The goal of humanisation is a reduction in the immunogenicity of a xenogenic antibody, such as a murine anti-hPG antibody, for introduction into a human, while maintaining the full antigen binding affinity and specificity of the antibody. The humanised antibodies or fragments of same can be prepared by techniques known to a person skilled in the art (such as, for example, those described in the documents Singer et al., J. Immun., 150:2844-2857, 1992). Such humanised antibodies are preferred for their use in methods involving *in vitro* diagnoses or preventive and/or therapeutic treatment *in vivo.* Other humanisation techniques are also known to the person skilled in the art. Indeed, Antibodies can be humanised using a variety of techniques including CDR- grafting (EP 0 451 261; EP 0 682 040; EP 0 939 127; EP 0 566 647; US 5,530,101; US 6,180,370; US 5,585,089; US 5,693,761; US 5,639,641; US 6,054,297; US 5,886,152; and US 5,877,293), veneering or resurfacing (EP 0 592 106; EP 0 519 596; Padlan E. A., 1991 , Molecular Immunology 28(4/5): 489-498; Studnicka G. M. et al., 1994, Protein Engineering 7(6): 805-814; Roguska M.A. et al., 1994, Proc. Natl. Acad. ScL U.S.A., 91:969-973), and chain shuffling (U.S. Pat. No. 5,565,332). Human antibodies can be made by a variety of methods known in the art including phage display methods. See also U.S. Pat. Nos. 4,444,887, 4,716,111, 5,545,806, and 5,814,318; and international patent application publication numbers WO 98/46645, WO 98/50433, WO 98/24893, WO 98/16654, WO 96/34096, WO 96/33735, and WO 91/10741.

In a more particular instance, said antibody is a humanised antibody selected in the group consisting of:
- A humanised antibody comprising a heavy chain comprising at least one, preferentially at least two, preferentially three, of CDR-H1, CDR-H2 and CDR-H3 of amino acid sequences SEQ ID N°4, 5 and 6, respectively, or sequences with at least 80%, preferably 85%, 90%, 95% and 98% identity after optimal alignment with sequences SEQ ID N°4, 5 and 6, respectively, and a light chain comprising at least one, preferentially at least two, preferentially three, of CDR-L1, CDR-L2 and CDR-L3 of amino acid sequences SEQ ID N°7, 8 and 9, respectively, or sequences with at least 80%, preferably 85%, 90%, 95% and 98% identity after optimal alignment with sequences SEQ ID N°7, 8 and 9, respectively,

- A humanised antibody comprising a heavy chain comprising at least one, preferentially at least two, preferentially three, of CDR-H1, CDR-H2 and CDR-H3 of amino acid sequences SEQ ID *N°10,* 11 and 12, respectively, or sequences with at least 80%, preferably 85%, 90%, 95% and 98% identity after optimal alignment with sequences SEQ ID *N°10,* 11 and 12, respectively, and a light chain comprising at least one, preferentially at least two, preferentially three, of CDR-L1, CDR-L2 and CDR-L3 of amino acid sequences SEQ ID N°13, 14 and 15, respectively, or sequences with at least 80%, preferably 85%, 90%, 95% and 98% identity after optimal alignment with sequences SEQ ID N° 13, 14 and 15, respectively,
- A humanised antibody comprising a heavy chain comprising at least one, preferentially at least two, preferentially three, of CDR-H1, CDR-H2 and CDR-H3 of amino acid sequences SEQ ID N°16, 17 and 18, respectively, or sequences with at least 80%, preferably 85%, 90%, 95% and 98% identity after optimal alignment with sequences SEQ ID N°16, 17 and 18, respectively, and a light chain comprising at least one, preferentially at least two, preferentially three, of CDR-L1, CDR-L2 and CDR-L3 of amino acid sequences SEQ ID N°19, 20 and 21, respectively, or sequences with at least 80%, preferably 85%, 90%, 95% and 98% identity after optimal alignment with sequences SEQ ID N° 19, 20 and 21, respectively,
- A humanised antibody comprising a heavy chain comprising at least one, preferentially at least two, preferentially three, of CDR-H1, CDR-H2 and CDR-H3 of amino acid sequences SEQ ID N°22, 23 and 24, respectively, or sequences with at least 80%, preferably 85%, 90%, 95% and 98% identity after optimal alignment with sequences SEQ ID N°22, 23 and 24, respectively, and a light chain comprising at least one, preferentially at least two, preferentially three, of CDR-L1, CDR-L2 and CDR-L3 of amino acid sequences SEQ ID N° 25, 26 and 27, respectively, or sequences with at least 80%, preferably 85%, 90%, 95% and 98% identity after optimal alignment with sequences SEQ ID N°25, 26 and 27, respectively,
- A humanised antibody comprising a heavy chain comprising at least one, preferentially at least two, preferentially three, of CDR-H1, CDR-H2 and CDR-H3 of amino acid sequences SEQ ID N°28, 29 and 30, respectively, or sequences with at least 80%, preferably 85%, 90%, 95% and 98% identity after optimal alignment with sequences SEQ ID N°28, 29 and 30, respectively, and a light chain comprising at least one, preferentially at least two, preferentially three, of CDR-L1, CDR-L2 and CDR-L3 of amino acid sequences SEQ ID N°31, 32 and 33, respectively, or sequences with at least 80%, preferably 85%, 90%, 95% and 98% identity after optimal alignment with sequences SEQ ID N°31, 32 and 33, respectively, and
- A humanised antibody comprising a heavy chain comprising at least one, preferentially at least two, preferentially three, of CDR-H1, CDR-H2 and CDR-H3 of amino acid sequences SEQ ID N°34, 35 and 36, respectively, or sequences with at least 80%, preferably 85%, 90%, 95% and 98% identity after optimal alignment with sequences SEQ ID N°34, 35 and 36, respectively, and a light chain comprising at least one, preferentially at least two, preferentially three, of CDR-L1, CDR-L2 and CDR-L3 of amino acid sequences SEQ ID N° 37, 38 and 39, respectively, or sequences with at least 80%, preferably 85%, 90%, 95% and 98% identity after optimal alignment with sequences SEQ ID N°37, 38 and 39, respectively,
wherein said antibody also comprises constant regions of the light-chain and the heavy-chain derived from a human antibody.

In another more particular instance, said antibody is a humanised antibody selected in the group consisting of:
- A humanised antibody comprising a heavy chain variable region of amino acid sequence SEQ ID N°53, and a light chain variable region of amino acid sequence SEQ ID N°54;
- A humanised antibody comprising a heavy chain variable region of amino acid sequence SEQ ID N°55, and a light chain variable region of amino acid sequence SEQ ID N°56;
- A humanised antibody comprising a heavy chain variable region of amino acid sequence selected between SEQ ID N°57, 58, and 59, and a light chain variable region of amino acid sequence selected between SEQ ID N°60, 61, and 62;

- A humanised antibody comprising a heavy chain variable region of amino acid sequence selected between SEQ ID N°63, 64, and 65, and a light chain variable region of amino acid sequence selected between SEQ ID N°66, 67, and 68;
- A humanised antibody comprising a heavy chain variable region of amino acid sequence selected between SEQ ID N°69 and 71, and a light chain variable region of amino acid sequence selected between SEQ ID N°70 and 72; and
- A humanised antibody comprising a heavy chain variable region of amino acid sequence selected between SEQ ID N°75 and 76, and a light chain variable region of amino acid sequence selected between SEQ ID N°77 and 78;
wherein said antibody also comprises constant regions of the light-chain and the heavy-chain derived from a human antibody.

Also included herein are anti-hPG antibodies which are derivatised, covalently modified, or conjugated to other molecules, for use in diagnostic and therapeutic applications. For example, but not by way of limitation, derivatised antibodies include antibodies that have been modified, *e.g.,* by glycosylation, acetylation, pegylation, phosphorylation, amidation, derivatisation by known protecting/blocking groups, proteolytic cleavage, linkage to a cellular ligand or other protein, *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-classical amino acids.

In another example, antibodies of the present disclosure can be attached to poly(ethyleneglycol) (PEG) moieties. In a specific example, the antibody is an antibody fragment and the PEG moieties are attached through any available amino acid sidechain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. See, for example U.S. Patent No. 5,219,996. Multiple sites can be used to attach two or more PEG molecules. PEG moieties can be covalently linked through a thiol group of at least one cysteine residue located in the antibody fragment. Where a thiol group is used as the point of attachment, appropriately activated effector moieties, for example thiol selective derivatives such as maleimides and cysteine derivatives, can be used.

In a specific example, an anti-hPG antibody conjugate is a modified Fab' fragment which is PEGylated, *i.e.,* has PEG (poly(ethyleneglycol)) covalently attached thereto, e.g., according to the method disclosed in EP0948544. See also Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D.C., 1997); and Bioconjugation Protein Coupling Techniques for the Biomedical Sciences, (M. Aslam and A. Dent, eds., Grove Publishers, New York, 1998); and Chapman, 2002, Advanced Drug Delivery Reviews 54:531-545. PEG can be attached to a cysteine in the hinge region. In one example, a PEG-modified Fab' fragment has a maleimide group covalently linked to a single thiol group in a modified hinge region. A lysine residue can be covalently linked to the maleimide group and to each of the amine groups on the lysine residue can be attached a methoxypoly(ethyleneglycol) polymer having a molecular weight of approximately 20,000 Da. The total molecular weight of the PEG attached to the Fab' fragment can therefore be approximately 40,000 Da.

Anti-hPG antibodies include labelled antibodies, useful in diagnostic applications. The antibodies can be used diagnostically, for example, to detect expression of a target of interest in specific cells, tissues, or serum; or to monitor the development or progression of an immunologic response as part of a clinical testing procedure to, *e.g.,* determine the efficacy of a given treatment regimen. Detection can be facilitated by coupling the antibody to a detectable substance or "label." A label can be conjugated directly or indirectly to an anti-hPG antibody of the disclosure. The label can itself be detectable (*e.g.,* radioisotope labels, isotopic labels, or fluorescent labels) or, in the case of an enzymatic label, can catalyse chemical alteration of a substrate compound or composition which is detectable. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance can be coupled or conjugated either directly to the antibody (or fragment thereof) or indirectly, through an intermediate (such as, for example, a linker known in the art) using techniques known in the art. Examples of enzymatic labels include luciferases (*e.g.,* firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, acetylcholinesterase, glucoamylase, lysozyme, saccharide oxidases (*e.g.,* glucose oxidase, galactose oxidase, and glucose-6-phosphate dehydrogenase), heterocyclic oxidases (such as uricase and xanthine oxidase), lactoperoxidase, microperoxidase, and the like. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, dimethylamine-1-napthalenesulfonyl chloride, or phycoerythrin and the like; an example of a luminescent material includes luminol; examples of bioluminescent materials include luciferase, luciferin, and aequorin; examples of suitable isotopic materials include ¹³C, ¹⁵N, and deuterium; and examples of suitable radioactive material include ¹²⁵I, ¹³¹I, ¹¹¹In or ⁹⁹Tc.

### Detection of progastrin using anti-hPG antibodies

Progastrin-binding molecules, such as e.g., anti-PG antibodies, are useful for applications that depend on PG detection such as identifying subjects susceptible to respond to immune checkpoint inhibitor therapy. Accordingly, the progastrin-binding molecules, including anti-PG antibodies, can be used in any of the methods described herein. Generally, said methods comprise measuring progastrin in a sample obtained from a patient using the anti-hPG antibodies of the disclosure, wherein a measurement of at least 3 pM, at least 5 pM, at least 10 pM, at least 20 pM, at least 30 pM, of progastrin in the sample is indicative of an absence of responsivity to immune checkpoint inhibitor therapy. Progastrin can be measured in samples of, e.g., blood, serum, plasma, tissue, and/or cells. hPG detection can be carried out using assays known in the art and/or described herein, such as, ELISA, sandwich ELISA, immunoblotting (Western blotting), immunoprecipitation, BIAcore technology and the like.

As noted herein, progastrin is but one of a number of different polypeptides resulting from post-translational processing of the gastrin gene product. Diagnostic, monitoring and other methods described herein specifically detect hPG as opposed to other gastrin gene products, including degradation products. The levels of progastrin can be measured by any method known to the person of skill in the art.

Preferably, determining the levels of progastrin in a sample includes contacting said sample with a progastrin-binding molecule and measuring the binding of said progastrin-binding molecule to progastrin.

When expression levels are measured at the protein level, it may be notably performed using specific progastrin-binding molecules, such as e.g., antibodies, in particular using well known technologies such as cell membrane staining using biotinylation or other equivalent techniques followed by immunoprecipitation with specific antibodies, western blot, ELISA or ELISPOT, enzyme-linked immunosorbant assays (ELISA), radioimmunoassays (RIA), immunohistochemistry (IHC), immunofluorescence (IF), antibodies microarrays, or tissue microarrays coupled to immunohistochemistry. Other suitable techniques include FRET or BRET, single cell microscopic or histochemistry methods using single or multiple excitation wavelength and applying any of the adapted optical methods, such as electrochemical methods (voltametry and amperometry techniques), atomic force microscopy, and radio frequency methods, e.g. multipolar resonance spectroscopy, confocal and nonconfocal, detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry), cell ELISA, flow cytometry, radioisotopic, magnetic resonance imaging, analysis by polyacrylamide gel electrophoresis (SDS-PAGE); HPLC-Mass Spectroscopy; Liquid Chromatography/Mass Spectrometry/Mass Spectrometry (LC-MS/MS)). All these techniques are well known in the art and need not be further detailed here. These different techniques can be used to measure the progastrin levels.

The progastrin-binding molecules described herein, especially the antiprogastrin antibodies, are particularly useful in an immunoassay. The immunoassay may be an enzyme-linked immunosorbent assay (ELISA), a radioimmunoassay (RIA), an immunodiffusion assay, or an immuno-detection assay, such as a surface plasmon resistance assay (e.g. a Biacore^{®} assay), an ELISPOT, slot-blot, or a western blot. As a general guide to such techniques, see for instance, Ausubel et al. (eds) (1987) in "Current Protocols in Molecular Biology" John Wiley and Sons, New York, N.Y.

Antibodies are key reagents in numerous assay techniques used in medical, veterinary and other immunodetection fields. Such tests include many routinely used immunoassay techniques, such as for example, enzyme-linked ELISA, RIA, IHC, and IF assays. The level of progastrin is preferentially assayed by any method known to one of skill in the art using antibodies directed against said protein. Preferably, the level of progastrin is determined using an immunoenzymatic assay, preferably based on techniques chosen between RIA and ELISA, with at least one progastrin-binding molecule. Most preferably, said level is determined by ELISA with at least one progastrin-binding molecule. More preferably, the level of progastrin is measured with one progastrin-binding molecule, using an immunoenzymatic assay, most preferably an ELISA assay.

In a particularly useful instance, the methods disclosed herein comprise determining the level of progastrin in a biological sample from a subject using an immunoenzymatic assay, preferably based on techniques chosen between RIA and ELISA, with a progastrin-binding molecule.

In general, the ELISA procedure for determining hPG levels using anti-hPG antibodies is as follows. A surface, such as the wells in a 96-well plate, is prepared to which a known quantity of a first, "capture," antibody to hPG is bound. The capture antibody can be, for example, an anti-hPG antibody which binds with the C- or N-terminus of hPG. After blocking, a test sample is applied to the surface followed by an incubation period. The surface is then washed to remove unbound antigen and a solution containing a second, "detection," antibody to hPG is applied. The detection antibody can be any of the anti-hPG antibodies described herein, provided the detection antibody binds a different epitope from the capture antibody. For example, if the capture antibody binds a C-terminal peptide region of hPG, then a suitable detection antibody would be one that binds an N-terminal peptide region of hPG. Alternatively, if the capture antibody binds a N-terminal peptide region of hPG, then a suitable detection antibody would be one that binds a C-terminal peptide region of hPG. Progastrin levels can then be detected either directly (if, for example, the detection antibody is conjugated to a detectable label) or indirectly (through a labelled secondary antibody that binds the detection anti-hPG antibody).

Specifically, hPG levels are measured as follows from a test sample. 96-well microtiter plates are coated with between 0.5 and 10 µg/mL of a rabbit C-terminal anti-hPG polyclonal antibody and incubated overnight. Plates are then washed three times in PBS-Tween (0.05%) and blocked with 2% (w/v) non-fat dried milk in PBS-Tween (0.05%). Separately, test samples, control samples (blank or PG-negative plasma or serum samples), and between about 5 pM (0.5 x 10-11 M) and about 0.1 nM (1x10-10 M) of an hPG reference standard (lyophilised hPG diluted in PG-negative plasma or serum) are prepared in an appropriate diluent (e.g., PBS-Tween 0.05%). Samples are incubated on the coated plates for between 2 and 4 hours at 37°C, or alternatively between 12 and 16 hours at 21°C. After incubation, plates are washed three times with PBS-Tween (0.05%) and incubated with between 0.001 and 0.1 µg/mL of an N-terminal anti-hPG monoclonal antibody as described herein, coupled to horseradish peroxidase (HRP) (Nakane et al., 1974, J. Histochem. Cytochem. 22(12): 1084-1091) for 30 minutes at 21°C. Plates are then washed three times in PBS-Tween (0.05%) and HRP substrate is added for 15 minutes at 21°C. The reaction is stopped by added 100 µL of 0.5M sulfuric acid and an optical density measurement taken at 405 nm. Test sample hPG levels are determined by comparison to a standard curve constructed from the measurements derived from the hPG reference standard.

In a first instance, a method disclosed herein comprises contacting a biological sample with an anti-hPG antibody binding to an epitope of hPG, wherein said epitope is located within the C-terminal part of hPG or to an epitope located within the N-terminal part of hPG.

More specifically, a method disclosed herein comprises contacting a biological sample with an anti-hPG antibody binding to an epitope of hPG, wherein said epitope includes an amino acid sequence corresponding to an amino acid sequence of the N-terminal part of progastrin chosen among an amino acid sequence corresponding to amino acids 10 to 14 of hPG, amino acids 9 to 14 of hPG, amino acids 4 to 10 of hPG, amino acids 2 to 10 of hPG and amino acids 2 to 14 of hPG, wherein the amino acid sequence of hPG is SEQ ID N°1.

More specifically, a method disclosed herein comprises contacting a biological sample with an anti-hPG antibody binding to an epitope of hPG, wherein said epitope includes an amino acid sequence corresponding to an amino acid sequence of the C-terminal part of progastrin, chosen among an amino acid sequence corresponding to amino acids 71 to 74 of hPG, amino acids 69 to 73 of hPG, amino acids 71 to 80 of hPG (SEQ ID N°40), amino acids 76 to 80 of hPG, and amino acids 67 to 74 of hPG, wherein the amino acid sequence of hPG is SEQ ID N°1.

In particular, the method of detecting PG comprises a step of contacting a biological sample from a subject with a first molecule which binds to a first part of progastrin and with a second molecule which binds to a second part of progastrin. More particularly, wherein said progastrin-binding molecule is an antibody, a biological sample from a subject is contacted with an antibody which binds to a first epitope of progastrin and with a second antibody which binds to a second epitope of progastrin.

Preferably, said first antibody is bound to an insoluble or partly soluble carrier. Binding of progastrin by said first antibody results in capture of progastrin from said biological sample. Preferably, said first antibody is an antibody binding to an epitope of hPG, wherein said epitope includes an amino acid sequence corresponding to an amino acid sequence of the C-terminal part of progastrin, as described above. More preferably, said first antibody is monoclonal antibody Mab14, produced by hybridoma 2H9F4B7, described in WO 2011/083088. Hybridoma 2H9F4B7 was deposited under the Budapest Treaty at the CNCM, Institut Pasteur, 25-28 rue du Docteur Roux, 75724 Paris CEDEX 15, France, on 27 December 2016, under reference I-5158.

Preferably, said second antibody is labelled with a detectable moiety, as described below. Binding of progastrin by second antibody enables the detection of the progastrin molecules which were present in the biological sample. Further, binding of progastrin by second antibody enables the quantification of the progastrin molecules which were present in the biological sample. Preferably, said second antibody is an antibody binding to an epitope of hPG, wherein said epitope includes an amino acid sequence corresponding to an amino acid sequence of the N-terminal part of progastrin, as described above. More preferably, said N-terminal antibody is a polyclonal antibody, as described above. Alternatively, it is also possible to use a monoclonal antibody biding an epitope within the N-terminus of progastrin, such as e.g. the N-terminus monoclonal antibodies described above, notably a monoclonal antibody comprising a heavy chain comprising CDR-H1, CDR-H2 and CDR-H3 of amino acid sequences SEQ ID N° 16, 17 and 18, respectively, and a light chain comprising CDR-L1, CDR-L2 and CDR-L3 of amino acid sequences SEQ ID N°19, 20 and 21.

More preferably, the first antibody is bound to an insoluble or partly soluble carrier and the second antibody is labelled with a detectable moiety.

Preferably, the method of the present disclosure for the diagnosis of lung cancer comprises the detection of progastrin in a biological sample from a human subject.

### Immune checkpoint inhibitors

In a first instance, the immune checkpoint inhibitor is an inhibitor of any one of CTLA-4, PDL1, PDL2, PD1, B7-H3, B7-H4, BTLA, HVEM, TIM3, GAL9, LAG3, VISTA, KIR, 2B4 (belongs to the CD2 family of molecules and is expressed on all NK, γδ, and memory CD8+ (αβ) T cells), CD 160 (also referred to as BY55), CGEN-15049, CHK 1 and CHK2 kinases, IDO1, A2aR and any of the various B-7 family ligands.

Exemplary immune checkpoint inhibitors include anti-CTLA-4 antibody (e.g., ipilimumab), anti-LAG-3 antibody (e.g., BMS-986016), anti-B7-H3 antibody, anti-B7-H4 antibody, anti-Tim3 antibody (e.g., TSR-022, MBG453), anti-BTLA antibody, anti-KIR antibody, anti-A2aR antibody, anti CD200 antibody, anti-PD-1 antibody (e.g., pembrolizumab, nivolumab, cemiplimab, pidilizumab), anti-PD-L1 antibody (e.g., atezolizumab, avelumab, durvalumab, BMS 936559), anti-VISTA antibody (e.g., JNJ 61610588), anti-CD28 antibody, anti-CD80 or -CD86 antibody, anti-B7RP1 antibody, anti-B7-H3 antibody, anti-HVEM antibody, anti-CD137 antibody (e.g., urelumab), anti-CD137L antibody, anti-OX40 (e.g., 9B12, PF-04518600, MEDI6469), anti-OX40L antibody, anti-CD40 or -CD40L antibody, anti-GAL9 antibody, anti-IL-10 antibody, fusion protein of the extracellular domain of a PD-1 ligand, e.g. PDL-1 or PD-L2, and IgG1 (e.g., AMP-224), fusion protein of the extracellular domain of a OX40 ligand, e.g. OX40L, and IgG1 (e.g., MEDI6383), IDO1 drug (e.g., epacadostat) and A2aR drug. A number of immune checkpoint inhibitors have been approved or are currently in clinical trials. Such inhibitors include ipilimumab, pembrolizumab, nivolumab, cemiplimab, pidilizumab, atezolizumab, avelumab, durvalumab, BMS 936559, JNJ 61610588, urelumab, 9B12, PF-04518600, BMS-986016, TSR-022, MBG453, MEDI6469, MEDI6383, and epacadostat.

Examples of immune checkpoints inhibitors are listed for example in Marin-Acevedo et al., Journal of Hematology & Oncology 11: 8, 2018; Kavecansky and Pavlick, AJHO 13(2): 9-20, 2017; Wei et al., Cancer Discov 8(9): 1069-86, 2018.

Preferably, the immune checkpoint inhibitor is an inhibitor of CTLA-4, LAG-3, Tim3, PD-1, PD-L1, VISTA, CD137, OX40, or IDO1.

In some instances, the inhibitor is a small molecule drug. In some instances, the inhibitor is a soluble receptor. In some instances, the inhibitor is an antibody.

In some instance, the inhibitor is an antagonistic antibody, i.e. an antibody that inhibits or reduces one or more of the biological activities of an antigen, such as any one of the immune checkpoint proteins described herein. Certain antagonistic antibodies substantially or completely inhibit one or more of the biological activities of said antigen. The term "inhibit," or a grammatical equivalent thereof, when used in the context of an antibody refers to an antibody that suppresses, restrains or decreases a biological activity of the antigen to which the antibody binds. The inhibitory effect of an antibody can be one which results in a measurable change in the antigen's biological activity.

In an instance, the immune checkpoint inhibitor is selected in the group consisting of ipilimumab, pembrolizumab, nivolumab, cemiplimab, pidilizumab, atezolizumab, avelumab, durvalumab, BMS 936559, JNJ 61610588, urelumab, 9B12, PF-04518600, BMS-986016, TSR-022, MBG453, MEDI6469, MEDI6383, and epacadostat.

In an instance, the immune checkpoint inhibitor is an inhibitor of CTLA-4, PD-1, or PD-L1. In a preferred instance, said immune checkpoint inhibitor is an antibody against any one of CTLA-4, PD-1, or PD-L1. More preferably, said antibody is an antagonist antibody. Even more preferably, said antagonist antibody is selected between ipilimumab, pembrolizumab, nivolumab, cemiplimab, pidilizumab, atezolizumab, avelumab, and durvalumab.

In an instance, the immune checkpoint inhibitor is an inhibitor of PD-1. In a preferred instance, said immune checkpoint inhibitor is an antibody against PD-1. More preferably, said antibody is an antagonist antibody. Even more preferably, the immune checkpoint inhibitor is pembrolizumab, nivolumab, cemiplimab, or pidilizumab.

### Nucleic acids and expression systems

The present disclosure encompasses polynucleotides encoding immunoglobulin light and heavy chain genes for antibodies, notably anti-hPG antibodies, vectors comprising such nucleic acids, and host cells capable of producing the antibodies of the disclosure.

In a first aspect, the present disclosure relates to one or more polynucleotides encoding an antibody, notably an antibody capable of binding specifically to progastrin as described above.

A first instance provides a polynucleotide encoding the heavy chain of an anti-hPG antibody described above. Preferably, said heavy chain comprises three heavy-chain CDRs of sequence SEQ ID NOS. 4, 5 and 6. More preferably, said heavy chain comprises a heavy chain comprising the variable region of sequence SEQ ID NO. 14. Even more preferably, said heavy chain has a complete sequence SEQ ID NO. 16.

In another instance, the polynucleotide encodes the light chain of an anti-hPG antibody described above. Preferably, said heavy chain comprises three heavy-chain CDRs of sequence SEQ ID NOS. 7, 8 and 9. More preferably, said heavy chain comprises a heavy chain comprising the variable region of sequence SEQ ID NO. 15. Even more preferably, said heavy chain has a complete sequence SEQ ID NO. 17.

A variety of expression systems may be used to express the antibody disclosed herein. In one aspect, such expression systems represent vehicles by which the coding sequences of interest may be produced and subsequently purified, but also represent cells which may, when transiently transfected with the appropriate nucleotide coding sequences, express an IgG antibody in situ.

The disclosure provides vectors comprising the polynucleotides described above. In one instance, the vector contains a polynucleotide encoding a heavy chain of the antibody of interest (e.g., an anti-hPG antibody). In another instance, said polynucleotide encodes the light chain of the antibody of interest (e.g., an anti-hPG antibody). The disclosure also provides vectors comprising polynucleotide molecules encoding fusion proteins, modified antibodies, antibody fragments, and probes thereof.

In order to express the heavy and/or light chain of the antibody of interest (e.g., an anti-hPG antibody), the polynucleotides encoding said heavy and/or light chains are inserted into expression vectors such that the genes are operatively linked to transcriptional and translational sequences.

"Operably linked" sequences include both expression control sequences that are contiguous with the gene of interest and expression control sequences that act in trans or at a distance to control the gene of interest. The term "expression control sequence" as used herein refers to polynucleotide sequences which are necessary to affect the expression and processing of coding sequences to which they are ligated. Expression control sequences include appropriate transcription initiation, termination, promoter and enhancer sequences; efficient RNA processing signals such as splicing and polyadenylation signals; sequences that stabilise cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

Certain vectors are capable of directing the expression of genes to which they are operatively linked. Such vectors are referred to herein as "recombinant expression vectors" (or simply, "expression vectors"). In general, expression vectors of utility in recombinant DNA techniques are in the form of plasmids. In the present specification, "plasmid" and "vector" may be used interchangeably as the plasmid is the most commonly used form of vector. However, the disclosure is intended to include such forms of expression vectors, such as bacterial plasmids, YACs, cosmids, retrovirus, EBV-derived episomes, and all the other vectors that the skilled man will know to be convenient for ensuring the expression of the heavy and/or light chains of the antibody of interest (e.g., an anti-hPG antibody). The skilled man will realise that the polynucleotides encoding the heavy and the light chains can be cloned into different vectors or in the same vector. Preferably, said polynucleotides are cloned into two vectors.

Polynucleotides disclosed herein and vectors comprising these molecules can be used for the transformation of a suitable host cell. The term "host cell", as used herein, is intended to refer to a cell into which a recombinant expression vector has been introduced in order to express the antibody of interest (e.g., an anti-hPG antibody). It should be understood that such terms are intended to refer not only to the particular subject cell but also to the progeny of such a cell. Because certain modifications may occur in succeeding generations due to either mutation or environmental influences, such progeny may not, in fact, be identical to the parent cell, but are still included within the scope of the term "host cell" as used herein.

Transformation can be performed by any known method for introducing polynucleotides into a cell host. Such methods are well known of the man skilled in the art and include dextran-mediated transformation, calcium phosphate precipitation, polybrene-mediated transfection, protoplast fusion, electroporation, encapsulation of the polynucleotide into liposomes, biolistic injection and direct microinjection of DNA into nuclei.

The host cell may be co-transfected with one or more expression vectors. For example, a host cell can be transfected with a vector encoding both the heavy chain and the light chain of the antibody of interest (e.g., an anti-hPG antibody), as described above. Alternatively, the host cell can be transformed with a first vector encoding the heavy chain of the antibody of interest (e.g., an anti-hPG antibody), and with a second vector encoding the light chain of said antibody. Mammalian cells are commonly used for the expression of a recombinant therapeutic immunoglobulins, especially for the expression of whole recombinant antibodies. For example, mammalian cells such as HEK293 or CHO cells, in conjunction with a vector, containing the expression signal such as one carrying the major intermediate early gene promoter element from human cytomegalovirus, are an effective system for expressing the humanised anti-hPG antibody disclosed herein (Foecking et al., 1986, Gene 45:101; Cockett et al., 1990, Bio/Technology 8: 2).

In addition, a host cell may be chosen which modulates the expression of the inserted sequences, or modifies and processes the gene product in the specific fashion desired. Such modifications (e.g., glycosylation) and processing of protein products may be important for the function of the protein. Different host cells have features and specific mechanisms for the post-translational processing and modification of proteins and gene products. Appropriate cell lines or host systems are chosen to ensure the correct modification and processing of the expressed antibody of interest. Hence, eukaryotic host cells which possess the cellular machinery for proper processing of the primary transcript, glycosylation of the gene product may be used. Such mammalian host cells include, but are not limited to, CHO, COS, HEK293, NS/0, BHK, Y2/0, 3T3 or myeloma cells (all these cell lines are available from public depositories such as the Collection Nationale des Cultures de Microorganismes, Paris, France, or the American Type Culture Collection, Manassas, VA, U.S.A.).

For long-term, high-yield production of recombinant proteins, stable expression is preferred. Cell lines which stably express the antibody may be engineered. Rather than using expression vectors which contain viral origins of replication, host cells are transformed with DNA under the control of the appropriate expression regulatory elements, including promoters, enhancers, transcription terminators, polyadenylation sites, and other appropriate sequences known to the person skilled in art, and a selectable marker. Following the introduction of the foreign DNA, engineered cells may be allowed to grow for one to two days in an enriched media, and then are moved to a selective media. The selectable marker on the recombinant plasmid confers resistance to the selection and allows cells to stably integrate the plasmid into a chromosome and be expanded into a cell line. Other methods for constructing stable cell lines are known in the art. In particular, methods for site-specific integration have been developed. According to these methods, the transformed DNA under the control of the appropriate expression regulatory elements, including promoters, enhancers, transcription terminators, polyadenylation sites, and other appropriate sequences is integrated in the host cell genome at a specific target site which has previously been cleaved (Moele et al., Proc. Natl. Acad. Sci. U.S.A., 104(9): 3055-3060; US 5,792,632; US 5,830,729; US 6,238,924; WO 2009/054985; WO 03/025183; WO 2004/067753).

A number of selection systems may be used according to the disclosure, including but not limited to the Herpes simplex virus thymidine kinase (Wigler et al., Cell 11:223, 1977), hypoxanthine-guanine phosphoribosyltransferase (Szybalska et al., Proc Natl Acad Sci USA 48: 202, 1992), glutamate synthase selection in the presence of methionine sulfoximide (Adv Drug Del Rev, 58: 671, 2006, and website or literature of Lonza Group Ltd.) and adenine phosphoribosyltransferase (Lowy et al., Cell 22: 817, 1980) genes in tk, hgprt or aprt cells, respectively. Also, antimetabolite resistance can be used as the basis of selection for the following genes: dhfr, which confers resistance to methotrexate (Wigler et al., Proc Natl Acad Sci USA 77: 357, 1980); gpt, which confers resistance to mycophenolic acid (Mulligan et al., Proc Natl Acad Sci USA 78: 2072, 1981); neo, which confers resistance to the aminoglycoside, G-418 (Wu et al., Biotherapy 3: 87, 1991); and hygro, which confers resistance to hygromycin (Santerre et al., Gene 30: 147, 1984). Methods known in the art of recombinant DNA technology may be routinely applied to select the desired recombinant clone, and such methods are described, for example, in Ausubel et al., eds., Current Protocols in Molecular Biology, John Wiley & Sons (1993). The expression levels of an antibody can be increased by vector amplification. When a marker in the vector system expressing an antibody is amplifiable, an increase in the level of inhibitor present in the culture will increase the number of copies of the marker gene. Since the amplified region is associated with the gene encoding the IgG antibody, production of said antibody will also increase (Crouse et al., Mol Cell Biol 3: 257, 1983). Alternative methods of expressing the gene exist and are known to the person of skills in the art. For example, a modified zinc finger protein can be engineered that is capable of binding the expression regulatory elements upstream of the gene; expression of the said engineered zinc finger protein (ZFN) in the host cell leads to increases in protein production (see e.g. Reik et al., Biotechnol. Bioeng., 97(5): 1180-1189, 2006). Moreover, ZFN can stimulate the integration of a DNA into a predetermined genomic location, resulting in high-efficiency site-specific gene addition (Moehle et al, Proc Natl Acad Sci USA, 104: 3055, 2007).

The antibody of interest (e.g., an anti-hPG antibody) may be prepared by growing a culture of the transformed host cells under culture conditions necessary to express the desired antibody. The resulting expressed antibody may then be purified from the culture medium or cell extracts. Soluble forms of the antibody of interest (e.g., an anti-hPG antibody) can be recovered from the culture supernatant. It may then be purified by any method known in the art for purification of an immunoglobulin molecule, for example, by chromatography (e.g., ion exchange, affinity, particularly by Protein A affinity for Fc, and so on), centrifugation, differential solubility or by any other standard technique for the purification of proteins. Suitable methods of purification will be apparent to a person of ordinary skills in the art.

Another aspect of the disclosure thus relates to a method for the production of an antibody (e.g., an anti-hPG antibody) described herein, said method comprising the steps of:
a) growing the above-described host cell in a culture medium under suitable culture conditions; and
b) recovering the antibody (e.g., an anti-hPG antibody), from the culture medium or from said cultured cells.

### Pharmaceutical compositions

The present immune checkpoint inhibitors can be formulated in compositions. Optionally, the compositions can comprise one or more additional therapeutic agents, such as the second therapeutic agents described below. The compositions will usually be supplied as part of a sterile, pharmaceutical composition that will normally include a pharmaceutically acceptable carrier and/or excipient. In another aspect, the disclosure thus provides a pharmaceutical composition comprising the immune checkpoint inhibitor and a pharmaceutical acceptable vehicle and/or an excipient.

This composition can be in any suitable form (depending upon the desired method of administering it to a patient). As used herein, "administering" is meant a method of giving a dosage of a compound (e.g., an immune checkpoint inhibitor, as described above) or a composition (e.g., a pharmaceutical composition, e.g., a pharmaceutical composition containing an immune checkpoint inhibitor, as described above) to a subject. The compositions utilised in the methods described herein can be administered, for example, intravitreally (e.g., by intravitreal injection), by eye drop, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumourally, peritoneally, subcutaneously, subconjunctivally, intravesicularly, mucosally, intrapericardially, intraumbilically, intraocularly, intraorbitally, orally, topically, transdermally, by inhalation, by injection, by implantation, by infusion, by continuous infusion, by localised perfusion bathing target cells directly, by catheter, by lavage, in cremes, or in lipid compositions. The compositions utilised in the methods described herein can also be administered systemically or locally. The method of administration can vary depending on various factors (e.g., the compound or composition being administered and the severity of the condition, disease, or disorder being treated). The most suitable route for administration in any given case will depend on the particular inhibitor, the subject, and the nature and severity of the disease and the physical condition of the subject. The immune checkpoint inhibitor can be formulated as an aqueous solution and administered by subcutaneous injection.

Pharmaceutical compositions can be conveniently presented in unit dose forms containing a predetermined amount of an immune checkpoint inhibitor per dose. Such a unit can contain for example but without limitation 5 mg to 5 g, for example 10 mg to 1 g, or 20 to 50 mg. Pharmaceutically acceptable carriers for use in the disclosure can take a wide variety of forms depending, e.g., on the condition to be treated or route of administration.

Pharmaceutical compositions of the disclosure can be prepared for storage as lyophilised formulations or aqueous solutions by mixing the antibody having the desired degree of purity with optional pharmaceutically-acceptable carriers, excipients or stabilisers typically employed in the art (all of which are referred to herein as "carriers"), *i.e.,* buffering agents, stabilising agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e.g.*, monosodium citrate-disodium citrate mixture, citric acid-trisodium citrate mixture, citric acid-monosodium citrate mixture, *etc.*)*,* succinate buffers (*e.g.,* succinic acid-monosodium succinate mixture, succinic acid-sodium hydroxide mixture, succinic acid-disodium succinate mixture, *etc*.)*,* tartrate buffers (*e.g.,* tartaric acid-sodium tartrate mixture, tartaric acid-potassium tartrate mixture, tartaric acid-sodium hydroxide mixture, *etc.*)*,* fumarate buffers (*e.g.,* fumaric acid-monosodium fumarate mixture, fumaric acid-disodium fumarate mixture, monosodium fumarate-disodium fumarate mixture, *etc*.), gluconate buffers (*e.g.,* gluconic acid-sodium glyconate mixture, gluconic acid-sodium hydroxide mixture, gluconic acid-potassium glyuconate mixture, *etc*.), oxalate buffer (*e.g.,* oxalic acid-sodium oxalate mixture, oxalic acid-sodium hydroxide mixture, oxalic acid-potassium oxalate mixture, *etc.*)*,* lactate buffers (*e.g.,* lactic acid-sodium lactate mixture, lactic acid-sodium hydroxide mixture, lactic acid-potassium lactate mixture, *etc.*) and acetate buffers (*e.g.,* acetic acid-sodium acetate mixture, acetic acid-sodium hydroxide mixture, *etc.*)*.* Additionally, phosphate buffers, histidine buffers and trimethylamine salts such as Tris can be used.

Preservatives can be added to retard microbial growth, and can be added in amounts ranging from 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (*e.g.*, chloride, bromide, and iodide), hexamethonium chloride, and alkyl parabens such as methyl or propyl paraben, catechol, resorcinol, cyclohexanol, and 3-pentanol. Isotonicifiers sometimes known as "stabilisers" can be added to ensure isotonicity of liquid compositions of the present disclosure and include polyhydric sugar alcohols, for example trihydric or higher sugar alcohols, such as glycerin, erythritol, arabitol, xylitol, sorbitol and mannitol. Stabilisers refer to a broad category of excipients which can range in function from a bulking agent to an additive which solubilises the therapeutic agent or helps to prevent denaturation or adherence to the container wall. Typical stabilisers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc.,* organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (*e.g.*, peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilisers can be present in the range from 0.1 to 10,000 weights per part of weight active protein.

Non-ionic surfactants or detergents (also known as "wetting agents") can be added to help solubilise the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, *etc*.), polyoxamers (184, 188, *etc*.), pluronic polyols, polyoxyethylene sorbitan monoethers (TWEEN^{®}-20, TWEEN^{®}-80, *etc*.). Non-ionic surfactants can be present in a range of about 0.05 mg/ml to about 1.0 mg/ml, for example about 0.07 mg/ml to about 0.2 mg/ml.

Additional miscellaneous excipients include bulking agents (*e.g.*, starch), chelating agents (*e.g.,* EDTA), antioxidants (*e.g.,* ascorbic acid, methionine, vitamin E), and cosolvents.

The present disclosure is further directed to a pharmaceutical composition comprising at least:
i) an immune checkpoint inhibitor and
ii) a second therapeutic agent, for example as described below,
as combination products for simultaneous, separate or sequential use.

"Simultaneous use" as used herein refers to the administration of the two compounds of the composition disclosed herein in a single and identical pharmaceutical form.

"Separate use" as used herein refers to the administration, at the same time, of the two compounds of the composition disclosed herein in distinct pharmaceutical forms.

"Sequential use" as used herein refers to the successive administration of the two compounds of the composition disclosed herein, each in a distinct pharmaceutical form.

Compositions of immune checkpoint inhibitors and a second therapeutic agents can be administered singly, as mixtures of one or more immune checkpoint inhibitors and/or one or more a second therapeutic agent, in mixture or combination with other agents useful for treating cancer, notably CRC, or adjunctive to other therapy for cancer, notably CRC. Examples of suitable combination and adjunctive therapies are provided below.

Encompassed by the present disclosure are pharmaceutical kits containing immune checkpoint inhibitors described herein. The pharmaceutical kit is a package comprising an immune checkpoint inhibitor (e.g., either in lyophilised form or as an aqueous solution) and one or more of the following:
- A second therapeutic agent, for example as described below;
- A device for administering the immune checkpoint inhibitor, for example a pen, needle and/or syringe; and
- Pharmaceutical grade water or buffer to resuspend the inhibitor if the inhibitor is in lyophilised form.

Each unit dose of the immune checkpoint inhibitor can be packaged separately, and a kit can contain one or more unit doses (*e.g.*, two unit doses, three unit doses, four unit doses, five unit doses, eight unit doses, ten unit doses, or more). Preferably, the one or more unit doses are each housed in a syringe or pen.

### Effective dosages

The immune checkpoint inhibitors will generally be used in an amount effective to achieve the intended result, for example an amount effective to treat cancer in a subject identified as displaying an immune checkpoint-inhibitor responsive phenotype by using any of the methods described above. Pharmaceutical compositions comprising immune checkpoint inhibitors can be administered to such patients (*e.g.*, human subjects) at therapeutically effective dosages.

The term "therapeutically effective dosage" means an amount of active compound or conjugate that elicits the desired biological response in a subject. Such response includes alleviation of the symptoms of the disease or disorder being treated, prevention, inhibition or a delay in the recurrence of symptom of the disease or of the disease itself, an increase in the longevity of the subject compared with the absence of the treatment, or prevention, inhibition or delay in the progression of symptom of the disease or of the disease itself. More specifically, a "therapeutically effective" dosage as used herein is an amount that confers a therapeutic benefit. A therapeutically effective dosage is also one in which any toxic or detrimental effects of the agent are outweighed by the therapeutically beneficial effects. In the context of CRC therapy, a therapeutic benefit means any amelioration of cancer, including any one of, or combination of, halting or slowing the progression of cancer (*e.g.*, from one stage of cancer to the next), halting or delaying aggravation or deterioration of the symptoms or signs of cancer, reducing the severity of cancer, inducing remission of cancer, inhibiting tumour cell proliferation, tumour size, or tumour number, or reducing PG serum levels.

Determination of the effective amount is well within the capability of those skilled in the art, especially in light of the detailed disclosure provided herein. Toxicity and therapeutic efficacy of a compound or a conjugate can be determined by standard pharmaceutical procedures in cell cultures and in experimental animals. The effective amount of present immune checkpoint inhibitors or other therapeutic agent to be administered to a subject will depend on the stage, category and status of the multiple myeloma and characteristics of the subject, such as general health, age, sex, body weight and drug tolerance. The effective amount of the present immune checkpoint inhibitors or other therapeutic agent to be administered will also depend on administration route and dosage form. Dosage amount and interval can be adjusted individually to provide plasma levels of the active compound that are sufficient to maintain desired therapeutic effects.

The amount of the immune checkpoint inhibitor administered will depend on a variety of factors, including the nature and stage of the cancer being treated, the form, route and site of administration, the therapeutic regimen (e.g., whether another therapeutic agent is used), the age and condition of the particular subject being treated, the sensitivity of the patient being treated to immune checkpoint inhibitors. The appropriate dosage can be readily determined by a person skilled in the art. Ultimately, a physician will determine appropriate dosages to be used. This dosage can be repeated as often as appropriate. If side effects develop the amount and/or frequency of the dosage can be altered or reduced, in accordance with normal clinical practice. The proper dosage and treatment regimen can be established by monitoring the progress of therapy using conventional techniques known to the people skilled of the art.

Effective dosages can be estimated initially from *in vitro* assays. For example, an initial dose for use in animals may be formulated to achieve a circulating blood or serum concentration of immune checkpoint inhibitor that is at or above the binding affinity of the inhibitor for the corresponding immune checkpoint protein as measured *in vitro.* Calculating dosages to achieve such circulating blood or serum concentrations taking into account the bioavailability of the particular inhibitor is well within the capabilities of skilled artisans. For guidance, the reader is referred to Fingl & Woodbury, "General Principles" in Goodman and Gilman's The Pharmaceutical Basis of Therapeutics, Chapter 1, latest edition, Pagamonon Press, and the references cited therein.

Initial dosages can be estimated from *in vivo* data, such as animal models. Animal models useful for testing the efficacy of compounds to treat each cancer type are well known in the art. Ordinarily skilled artisans can routinely adapt such information to determine dosages suitable for human administration.

The effective dose of an immune checkpoint inhibitor as described herein can range from about 0.001 to about 75 mg/kg per single (e.g., bolus) administration, multiple administrations or continuous administration, or to achieve a serum concentration of 0.01-5000 µg/ml serum concentration per single (e.g., bolus) administration, multiple administrations or continuous administration, or any effective range or value therein depending on the condition being treated, the route of administration and the age, weight and condition of the subject. In a certain instance, each dose can range from about 0.5 µg to about 50 µg per kilogram of body weight, for example from about 3 µg to about 30 µg per kilogram body weight.

Amount, frequency, and duration of administration will depend on a variety of factors, such as the patient's age, weight, and disease condition. A therapeutic regimen for administration can continue for 2 weeks to indefinitely, for 2 weeks to 6 months, from 3 months to 5 years, from 6 months to 1 or 2 years, from 8 months to 18 months, or the like. Optionally, the therapeutic regimen provides for repeated administration, *e.g.*, once daily, twice daily, every two days, three days, five days, one week, two weeks, or one month. The repeated administration can be at the same dose or at a different dose. The administration can be repeated once, twice, three times, four times, five times, six times, seven times, eight times, nine times, ten times, or more. A therapeutically effective amount of an immune checkpoint inhibitor can be administered as a single dose or over the course of a therapeutic regimen, *e.g.,* over the course of a week, two weeks, three weeks, one month, three months, six months, one year, or longer.

### Therapeutic methods

The methods disclosed herein are particularly useful for treating cancer, as they allow selecting patients who will respond to immunotherapy.

Accordingly, an aspect of the present disclosure thus relates to a method of treatment of cancer comprising administering an immune checkpoint inhibitor to a cancer patient, said method comprising a prior step of selecting a patient responsive to immune checkpoint inhibitors.

In another instance, the disclosure relates to an immune checkpoint inhibitor for use in treating cancer, wherein said use comprises a prior step of selecting a patient responsive to immune checkpoint inhibitors.

Accordingly, it is herein provided an immune checkpoint inhibitor for use in treating cancer, said use comprising:
a) selecting a patient responsive to immune checkpoint inhibitors using a method disclosed herein.

Another instance relates to the use of an immune checkpoint inhibitor for making a medicament for treating cancer, wherein said treatment comprises a prior step of selecting a patient responsive to immune checkpoint inhibitors.

Said patient selection is performed by any of the methods described above.

The disclosure also relates to a method for designing an immune checkpoint inhibitor treatment for a subject suffering from cancer, said method comprising:
a) determining the immune-checkpoint-inhibitor responding or non-responding phenotype according to the methods described above, and
b) designing the dose of immune checkpoint inhibitor treatment according to said identified immune-checkpoint-inhibitor responding or non-responding phenotype.

The present disclosure is also drawn to a method of treatment of a cancer-suffering subject with an immune checkpoint inhibitor, comprising:
a) determining from a biological sample of the said cancer-suffering subject the presence of an immune-checkpoint-inhibitor responding or non-responding phenotype using a method according to the disclosure, and
b) adapting the immune checkpoint inhibitor treatment in function of the result of step (a).

Optionally, the dose of immune checkpoint inhibitor determined in step (b) is administered to the subject.

The disclosure also relates to an immune checkpoint inhibitor for use in the treatment of cancer, said use comprising:
a) determining from a biological sample of the said cancer-suffering subject the presence of an immune-checkpoint-inhibitor responding or non-responding phenotype using a method disclosed herein, and
b) adapting the immune checkpoint inhibitor treatment in function of the result of step (a).

Optionally, the dose of immune checkpoint inhibitor determined in step (b) is administered to the subject.

Another instance relates to the use of an immune checkpoint inhibitor for making a medicament for the treatment of cancer, said treatment comprising:
a) determining from a biological sample of the said cancer-suffering subject the presence of an immune-checkpoint-inhibitor responding or non-responding phenotype using a method disclosed herein, and
b) adapting the immune checkpoint inhibitor treatment in function of the result of step (a).

Said adaptation of the immune-checkpoint-inhibitor treatment may consist in:
- a reduction or suppression of the said immune-checkpoint-inhibitor treatment if the subject has been identified as immune-checkpoint-inhibitor non-responding, or
- the continuation of the said immune-checkpoint-inhibitor treatment if the subject has been identified as immune-checkpoint-inhibitor responding.

The present disclosure thus provides methods of treating cancer in a patient in need thereof. Generally, the methods comprise administering to the patient a therapeutically effective amount of the immune checkpoint inhibitor described herein. In another instance, the present disclosure provides the immune checkpoint inhibitor described herein for use in the treatment of cancer. Examples of cancer which can be treated according to the methods disclosed herein include but are not limited to, carcinoma, lymphoma, blastoma, sarcoma, and leukaemia or lymphoid malignancies. More specifically, a cancer is selected from the group comprising squamous cell cancer (e.g., epithelial squamous cell cancer), lung cancer including small-cell lung cancer, non-small cell lung cancer, adenocarcinoma of the lung and squamous carcinoma of the lung, oropharyngeal cancer, nasopharyngeal cancer, laryngeal cancer, cancer of the peritoneum, oesophageal cancer, hepatocellular cancer, gastric or stomach cancer including gastrointestinal cancer and gastrointestinal stromal cancer, pancreatic cancer, glioblastoma, brain cancer, nervous system cancer, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, gallbladder cancer, vulval cancer, testicular cancer, thyroid cancer, Kaposi sarcoma, hepatic carcinoma, anal carcinoma, penile carcinoma, non-melanoma skin cancer, melanoma, skin melanoma, superficial spreading melanoma, lentigo maligna melanoma, acral lentiginous melanomas, nodular melanomas, multiple myeloma and B-cell lymphoma (including Hodgkin lymphoma; non-Hodgkin lymphoma, such as e.g., low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukaemia (CLL); acute lymphoblastic leukaemia (ALL); hairy cell leukaemia; chronic myeloblastic leukaemia (CML); Acute Myeloblastic Leukaemia (AML); and post-transplant lymphoproliferative disorder (PTLD), as well as abnormal vascular proliferation associated with phacomatoses, oedema (such as that associated with brain tumours), Meigs' syndrome, brain, as well as head and neck cancer, including lip & oral cavity cancer, and associated metastases.

Preferably, said cancer is lung cancer, lip & oral cavity cancer, oropharyngeal cancer, nasopharyngeal cancer, laryngeal cancer, prostate cancer, oesophageal cancer, gallbladder cancer, liver cancer, hepatocellular cancer, gastric or stomach cancer including gastrointestinal cancer and gastrointestinal stromal cancer, pancreatic cancer, Hodgkin lymphoma, Non-Hodgkin lymphoma, leukemia, multiple myeloma, Kaposi sarcoma, kidney cancer, bladder cancer, colon cancer, rectal cancer, colorectal cancer, hepatoma, hepatic carcinoma, anal carcinoma, thyroid cancer, non-melanoma skin cancer, skin melanoma, brain cancer, nervous system cancer, testicular cancer, cervical cancer, uterine cancer, endometrial cancer, ovarian cancer, or breast cancer.

More preferably, said cancer is oesophageal cancer, liver cancer, hepatocellular cancer, gastric or stomach cancer including gastrointestinal cancer and gastrointestinal stromal cancer, pancreatic cancer, Hodgkin lymphoma, colon cancer, rectal cancer, colorectal cancer, hepatoma, hepatic carcinoma, anal carcinoma, non-melanoma skin cancer, skin melanoma, cervical cancer, uterine cancer, endometrial cancer, ovarian cancer, or breast cancer.

The subject to whom the present immune checkpoint inhibitor is administered is preferably a mammal such as a non-primate (*e.g.*, cow, pig, horse, cat, dog, rat, *etc*.) or a primate (*e.g.,* monkey or human). The subject or patient is preferably a human, such as an adult patient or a paediatric patient.

Patients suitable for immune checkpoint inhibitor therapy are patients diagnosed with cancer. The cancer can be of any type and at any clinical stage or manifestation. Suitable subjects include patients with tumours (operable or inoperable), patients whose tumours have been surgically removed or resected, patients with a tumour comprising cells carrying a mutation in an oncogene, such as, for example, RAS or APC, patients who have received or receive other therapy for cancer in combination with or adjunctive to immune checkpoint inhibitor therapy. Other therapy for cancer includes, but is not limited to, chemotherapeutic treatment, radiation therapy, surgical resection, and treatment with one or more other therapeutic antibodies, as detailed below.

According to other instances, immune checkpoint inhibitors as disclosed herein are administered in a composition to a subject in need of prevention of metastatic cancer in a therapeutically effective amount. Such subjects include, but are not limited to those determined to have primary cancer but in whom the cancer is not known to have spread to distant tissues or organs.

According to yet other instances, the immune checkpoint inhibitors as disclosed herein are administered in a composition to a subject in need of prevention for recurrence of metastatic cancer in a therapeutically effective amount. Such subjects include, but are not limited to those who were previously treated for primary or metastatic cancer, after which treatment such cancer apparently disappeared.

According to other instances, immune checkpoint inhibitors as disclosed herein are administered in a composition to a subject in need of inhibition of the growth of cancer stem cells in a therapeutically effective amount. Such subjects include, but are not limited to those having a cancer the growth or metastasis of which is at least partly attributable to the presence within it of cancer stem cells. Other instances provide for methods of preventing or inhibiting the growth of cancer stem cells by contacting such stem cells with an amount of an immune checkpoint inhibitor composition effective to prevent or inhibit the growth of such cells. Such methods can be carried out *in vitro* or *in vivo.*

Serum PG levels are also useful in assessing efficacy of cancer treatment. Accordingly, the present disclosure provides a method for monitoring the effectiveness of cancer therapy with an immune checkpoint inhibitor comprising determining PG levels in a patient being treated for cancer with said inhibitor. Methods for monitoring the effectiveness of cancer therapy comprise repeatedly determining hPG levels using an anti-PG monoclonal antibody of the present disclosure in a cancer patient undergoing treatment for cancer, said treatment comprising the administration of an immune checkpoint inhibitor, wherein a decrease in the patient's circulating hPG levels over an interval of treatment is indicative of treatment efficacy. For example, a first measurement of a patient's circulating hPG levels can be taken followed by a second measurement while or after the patient receives treatment for colorectal cancer. The two measurements are then compared, and a decrease in hPG levels is indicative of therapeutic benefit.

An immune checkpoint inhibitor therapy can be combined with, or adjunctive to, one or more other treatments. Other treatments include, without limitation, chemotherapeutic treatment, radiation therapy, surgical resection, and antibody therapy, as described herein.

An immune checkpoint inhibitor therapy can be adjunctive to other treatment, including surgical resection.

Combination therapy as provided herein involves the administration of at least two agents to a patient, the first of which is an immune checkpoint inhibitor combination of the disclosure, and the second of which is another therapeutic agent. According to this instance, the disclosure relates to the immune checkpoint inhibitor described above, for the treatment of cancer, wherein said immune checkpoint inhibitor is administered with said other therapeutic agent. The immune checkpoint inhibitor and the other therapeutic agent can be administered simultaneously, successively, or separately.

A "therapeutic agent" encompasses biological agents, such as an antibody, a peptide, a protein, an enzyme, and chemotherapeutic agents. The therapeutic agent also encompasses immuno-conjugates of cell-binding agents (CBAs) and chemical compounds, such as antibody-drug conjugates (ADCs). The drug in the conjugates can be a cytotoxic agent, such as one described herein.

As used herein, the immune checkpoint inhibitor and the other therapeutic agent are said to be administered successively if they are administered to the patient on the same day, for example during the same patient visit. Successive administration can occur 1, 2, 3, 4, 5, 6, 7 or 8 hours apart. In contrast, the immune checkpoint inhibitor of the disclosure and the other therapeutic agent are said to be administered separately if they are administered to the patient on the different days, for example, the immune checkpoint inhibitor of the disclosure and the other therapeutic agent can be administered at a 1-day, 2-day or 3-day, one-week, 2-week or monthly intervals. In the methods of the present disclosure, administration of the immune checkpoint inhibitor of the disclosure can precede or follow administration of the other therapeutic agent.

As a non-limiting example, the instant immune checkpoint inhibitor and other therapeutic agent can be administered concurrently for a period of time, followed by a second period of time in which the administration of the immune checkpoint inhibitor of the disclosure and the other therapeutic agent is alternated.

Combination therapies of the present disclosure can result in a greater than additive, or a synergistic, effect, providing therapeutic benefits where neither the immune checkpoint inhibitor nor other therapeutic agent is administered in an amount that is, alone, therapeutically effective. Thus, such agents can be administered in lower amounts, reducing the possibility and/or severity of adverse effects.

Preferably, the other therapeutic agent is a chemotherapeutic agent. Said chemotherapeutic agent is preferably an alkylating agent, an anti-metabolite, an anti-tumour antibiotic, a mitotic inhibitor, a chromatin function inhibitor, an anti-angiogenesis agent, an anti-oestrogen, an anti-androgen or an immunomodulator.

The term "alkylating agent," as used herein, refers to any substance which can cross-link or alkylate any molecule, preferably nucleic acid (e.g., DNA), within a cell. Examples of alkylating agents include nitrogen mustard such as mechlorethamine, chlorambucol, melphalen, chlorydrate, pipobromen, prednimustin, disodic-phosphate or estramustine; oxazophorins such as cyclophosphamide, altretamine, trofosfamide, sulfofosfamide or ifosfamide; aziridines or imine-ethylènes such as thiotepa, triethylenamine or altetramine; nitrosourea such as carmustine, streptozocin, fotemustin or lomustine; alkyle-sulfonates such as busulfan, treosulfan or improsulfan; triazenes such as dacarbazine; or platinum complexes such as cis-platinum, oxaliplatin and carboplatin.

The expression "anti-metabolites," as used herein, refers to substances that block cell growth and/or metabolism by interfering with certain activities, usually DNA synthesis. Examples of anti-metabolites include methotrexate, 5-fluoruracil, floxuridine, 5-fluorodeoxyuridine, capecitabine, cytarabine, fludarabine, cytosine arabinoside, 6-mercaptopurine (6-MP), 6-thioguanine (6-TG), chlorodesoxyadenosine, 5-azacytidine, gemcitabine, cladribine, deoxycoformycin and pentostatin.

As used herein, "anti-tumour antibiotics" are compounds which may prevent or inhibit DNA, RNA and/or protein synthesis. Examples of anti-tumour antibiotics include doxorubicin, daunorubicin, idarubicin, valrubicin, mitoxantrone, dactinomycin, mithramycin, plicamycin, mitomycin C, bleomycin, and procarbazine.

"Mitotic inhibitors," as used herein, prevent normal progression of the cell cycle and mitosis. In general, microtubule inhibitors or taxoids such as paclitaxel and docetaxel are capable of inhibiting mitosis. Vinca alkaloid such as vinblastine, vincristine, vindesine and vinorelbine are also capable of inhibiting mitosis.

As used herein, the terms "chromatin function inhibitors" or "topoisomerase inhibitors" refer to substances which inhibit the normal function of chromatin modeling proteins such as topoisomerase I or topoisomerase II. Examples of chromatin function inhibitors include, for topoisomerase I, camptothecine and its derivatives such as topotecan or irinotecan, and, for topoisomerase II, etoposide, etoposide phosphate and teniposide.

As used herein, the term "anti-angiogenesis agent" refers to any drug, compound, substance or agent which inhibits growth of blood vessels. Exemplary anti-angiogenesis agents include, but are by no means limited to, razoxin, marimastat, batimastat, prinomastat, tanomastat, ilomastat, CGS-27023A, halofuginon, COL-3, neovastat, BMS-275291, thalidomide, CDC 501, DMXAA, L-651582, squalamine, endostatin, SU5416, SU6668, interferon-alpha, EMD121974, interleukin-12, IM862, angiostatin and vitaxin.

As used herein, the terms "anti-oestrogen" or "anti-estrogenic agent" refer to any substance which reduces, antagonizes or inhibits the action of estrogen. Examples of anti-oestrogen agents are tamoxifen, toremifene, raloxifene, droloxifene, iodoxyfene, anastrozole, letrozole, and exemestane.

As used herein, the terms "anti-androgens" or "anti-androgen agents" refer to any substance which reduces, antagonizes or inhibits the action of an androgen. Examples of anti-androgens are flutamide, nilutamide, bicalutamide, sprironolactone, cyproterone acetate, finasteride and cimitidine.

"Immunomodulators" as used herein are substances which stimulate the immune system.

Examples of immunomodulators include interferon, interleukin such as aldesleukine, OCT-43, denileukin diflitox and interleukin-2, tumoural necrose fators such as tasonermine or others immunomodulators such as lentinan, sizofiran, roquinimex, pidotimod, pegademase, thymopentine, poly I:C or levamisole in conjunction with 5-fluorouracil.

For more detail, the person of skill in the art can refer to the manual edited by the "Association Française des Enseignants de Chimie Thérapeutique" and entitled "Traité de chimie thérapeutique", vol. 6, Médicaments antitumouraux et perspectives dans le traitement des cancers, edition TEC & DOC, 2003.

It can also be mentioned as chemical agents or cytotoxic agents, all kinase inhibitors such as, for example, gefitinib or erlotinib.

More generally, examples of suitable chemotherapeutic agents include but are not limited to 1-dehydrotestosterone, 5-fluorouracil decarbazine, 6-mercaptopurine, 6-thioguanine, actinomycin D, adriamycin, aldesleukin, alkylating agents, allopurinol sodium, altretamine, amifostine, anastrozole, anthramycin (AMC)), anti-mitotic agents, cis-dichlorodiamine platinum (II) (DDP) cisplatin), diamino dichloro platinum, anthracyclines, antibiotics, antimetabolites, asparaginase, BCG live (intravesical), betamethasone sodium phosphate and betamethasone acetate, bicalutamide, bleomycin sulfate, busulfan, calcium leucouorin, calicheamicin, capecitabine, carboplatin, lomustine (CCNU), carmustine (BSNU), Chlorambucil, Cisplatin, Cladribine, Colchicin, conjugated estrogens, Cyclophosphamide, Cyclothosphamide, Cytarabine, Cytarabine, cytochalasin B, Cytoxan, Dacarbazine, Dactinomycin, dactinomycin (formerly actinomycin), daunirubicin HCL, daunorucbicin citrate, denileukin diftitox, Dexrazoxane, Dibromomannitol, dihydroxy anthracin dione, Docetaxel, dolasetron mesylate, doxorubicin HCL, dronabinol, *E. coli* L-asparaginase, emetine, epoetin-α, Erwinia L-asparaginase, esterified estrogens, estradiol, estramustine phosphate sodium, ethidium bromide, ethinyl estradiol, etidronate, etoposide citrororum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon α-2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptipurine, mesna, methotrexate, methyltestosterone, mithramycin, mitomycin C, mitotane, mitoxantrone, nilutamide, octreotide acetate, ondansetron HCL, oxaliplatin, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, tegafur, teniposide, tenoposide, testolactone, tetracaine, thioepa chlorambucil, thioguanine, thiotepa, topotecan HCL, toremifene citrate, trastuzumab, tretinoin, valrubicin, vinblastine sulfate, vincristine sulfate, and vinorelbine tartrate.

The immune checkpoint inhibitors disclosed herein can be administered to a patient in need of treatment for colorectal cancer receiving a combination of chemotherapeutic agents. Exemplary combinations of chemotherapeutic agents include 5-fluorouracil (5FU) in combination with leucovorin (folinic acid or LV); capecitabine, in combination with uracil (UFT) and leucovorin; tegafur in combination with uracil (UFT) and leucovorin; oxaliplatin in combination with 5FU, or in combination with capecitabine; irinotecan in combination with capecitabine, mitomycin C in combination with 5FU, irinotecan or capecitabine. Use of other combinations of chemotherapeutic agents disclosed herein is also possible.

As is known in the relevant art, chemotherapy regimens for colorectal cancer using combinations of different chemotherapeutic agents have been standardised in clinical trials. Such regimens are often known by acronyms and include 5FU Mayo, 5FU Roswell Park, LVFU2, FOLFOX, FOLFOX4, FOLFOX6, bFOL, FUFOX, FOLFIRI, IFL, XELOX, CAPOX, XELIRI, CAPIRI, FOLFOXIRI. *See, e.g.,* Chau, I., et al., 2009, Br. J. Cancer 100:1704-19 and Field, K., et al., 2007, World J. Gastroenterol. 13:3806-15.

Immune checkpoint inhibitors can also be combined with other therapeutic antibodies. Accordingly, immune checkpoint inhibitor therapy can be combined with, or administered adjunctive to a different monoclonal antibody such as, for example, but not by way of limitation, an anti-EGFR (EGF receptor) monoclonal antibody or an anti-VEGF monoclonal antibody. Specific examples of anti-EGFR antibodies include cetuximab and panitumumab. A specific example of an anti-VEGF antibody is bevacizumab.

According to this instance, the disclosure relates to the immune checkpoint inhibitor described above, for the treatment of cancer, wherein said inhibitor is administered with a chemotherapeutic agent. The immune checkpoint inhibitor and the chemotherapeutic agent can be administered simultaneously, successively, or separately.

### Diagnostic kits

In an aspect, the disclosure provides diagnostic kits containing the anti-PG antibodies (including antibody conjugates). The diagnostic kit is a package comprising at least one anti-PG antibody of the disclosure (e.g., either in lyophilised form or as an aqueous solution) and one or more reagents useful for performing a diagnostic assay (e.g., diluents, a labelled antibody that binds to an anti-PG antibody, an appropriate substrate for the labelled antibody, PG in a form appropriate for use as a positive control and reference standard, a negative control). In specific instances, a kit comprises two anti-PG antibodies, wherein at least one of the antibodies is an anti-PG monoclonal antibody. Optionally, the second antibody is a polyclonal anti-PG antibody. In some instances, the kit of the present disclosure comprises an N-terminal anti-PG monoclonal antibody as described herein.

Anti-PG antibodies can be labelled, as described above. In an instance, anti-PG antibodies or antigen-binding fragments thereof as detailed herein are provided labelled with a detectable moiety, such that they may be packaged and used, for example, in kits, to diagnose or identify cells having the aforementioned antigen. Non-limiting examples of such labels include fluorophores such as fluorescein isothiocyanate; chromophores, radionuclides, biotin or enzymes. Such labelled anti-PG antibodies may be used for the histological localization of the antigen, ELISA, cell sorting, as well as other immunological techniques for detecting or quantifying PG, and cells bearing this antigen, for example.

Alternatively, the kit can include a labelled antibody which binds an anti-PG monoclonal antibody and is conjugated to an enzyme. Where the anti-PG monoclonal antibody or other antibody is conjugated to an enzyme for detection, the kit can include substrates and cofactors required by the enzyme (e.g., a substrate precursor which provides the detectable chromophore or fluorophore). In addition, other additives can be included, such as stabilisers, buffers (e.g., a block buffer or lysis buffer), and the like. Anti-hPG monoclonal antibodies included in a diagnostic kit can be immobilised on a solid surface, or, alternatively, a solid surface (e.g., a slide) on which the antibody can be immobilised is included in the kit. The relative amounts of the various reagents can be varied widely to provide for concentrations in solution of the reagents which substantially optimise the sensitivity of the assay. Antibodies and other reagents can be provided (individually or combined) as dry powders, usually lyophilised, including excipients which on dissolution will provide a reagent solution having the appropriate concentration.

Kits may include instructional materials containing instructions (e.g., protocols) for the practice of diagnostic methods. While the instructional materials typically comprise written or printed materials, they are not limited to such. A medium capable of storing such instructions and communicating them to an end user is contemplated by this disclosure. Such media include, but are not limited to, electronic storage media (e.g., magnetic discs, tapes, cartridges, chips), optical media (e.g., CD ROM), and the like. Such media may include addresses to internet sites that provide such instructional materials.

### EXAMPLE

In this study, 43 plasma samples from patient having melanoma were tested for blood progastrin levels before starting treatment with immune checkpoint inhibitors therapy.

Each plasma EDTA sample was tested in duplicate using 50 µL plasma per well in an ELISA assay. Briefly, the assay utilises a capture antibody specific for hPG precoated on a 96-well plate. hPG is captured with the C-terminus monoclonal antibody mAb 14 produced by hybridoma 2H9F4B7 described in WO 2011/083088 (Hybridoma 2H9F4B7 is deposited under the Budapest Treaty at the CNCM, Institut Pasteur, 25-28 rue du Docteur Roux, 75724 Paris CEDEX 15, France, on 27 December 2016, under reference I-5158.). The hPG present in standards and samples added to the wells binds to the immobilised capture antibody. The wells are washed and a horseradish peroxidase (HRP) conjugated anti-hPG detection antibody added (detection is performed with labelled polyclonal antibodies specific for the N-terminus.), resulting in an antibody-antigen-antibody complex. After a second wash, a 3,3',5,5'-tetramethylbenzidine (TMB) substrate solution is added to the well, producing a blue color in direct proportion to the amount of hPG present in the initial sample. The Stop Solution changes the color from blue to yellow, and the intensity of the yellow color is quantified at 450 nm with a microplate reader.

These patients were separated in two groups: patients with a progastrin blood levels below 3 pM (n=21) and over 3 pM (n=22). Kaplan-Meier survival analyses were conducted in GraphPad by log-rank test (Mantel-Cox) and Gehan-Breslow-Wilcoxon test. The median survival of the group PG<3 pM and of the group PG>3 pM was 151 days and 68.5 days respectively showing an increase of 2.2 (95% CI of the ratio between 1.651 and 2.758) median survival for the patient with a low level of PG (PG<3pM).

## Claims

1. An *in vitro* method for selecting a melanoma patient susceptible to responding to treatment with an immune checkpoint inhibitor, said method comprising the steps of:
a) contacting a biological sample from said subject with at least one progastrin-binding monoclonal antibody,
wherein the biological sample is blood, plasma or serum,
and wherein the monoclonal antibody is selected in the group consisting of:
- A monoclonal antibody comprising a heavy chain comprising CDR-H1, CDR-H2 and CDR-H3 of amino acid sequences SEQ ID N°4, 5 and 6, respectively, and a light chain comprising CDR-L1, CDR-L2 and CDR-L3 of amino acid sequences SEQ ID N°7, 8 and 9, respectively,
- A monoclonal antibody comprising a heavy chain comprising CDR-H1, CDR-H2 and CDR-H3 of amino acid sequences SEQ ID *N°10,* 11 and 12, respectively, and a light chain comprising CDR-L1, CDR-L2 and CDR-L3 of amino acid sequences SEQ ID N°13, 14 and 15, respectively,
- A monoclonal antibody comprising a heavy chain comprising CDR-H1, CDR-H2 and CDR-H3 of amino acid sequences SEQ ID N°16, 17 and 18, respectively, and a light chain comprising CDR-L1, CDR-L2 and CDR-L3 of amino acid sequences SEQ ID N°19, 20 and 21, respectively,
- A monoclonal antibody comprising a heavy chain comprising CDR-H1, CDR-H2 and CDR-H3 of amino acid sequences SEQ ID N°22, 23 and 24, respectively, and a light chain comprising CDR-L1, CDR-L2 and CDR-L3 of amino acid sequences SEQ ID N°25, 26 and 27, respectively,
- A monoclonal antibody comprising a heavy chain comprising CDR-H1, CDR-H2 and CDR-H3 of amino acid sequences SEQ ID N°28, 29 and 30, respectively, and a light chain comprising CDR-L1, CDR-L2 and CDR-L3 of amino acid sequences SEQ ID N°31, 32 and 33, respectively,
- A monoclonal antibody comprising a heavy chain comprising CDR-H1, CDR-H2 and CDR-H3 of amino acid sequences SEQ ID N°34, 35 and 36, respectively, and a light chain comprising CDR-L1, CDR-L2 and CDR-L3 of amino acid sequences SEQ ID N°37, 38 and 39, respectively, and
- A monoclonal antibody produced by the hybridoma deposited at the CNCM, Institut Pasteur, 25-28 rue du Docteur Roux, 75724 Paris CEDEX 15, France, on 27 December 2016, under reference I-5158.
and
b) detecting the binding of said progastrin-binding molecule to progastrin in said sample, wherein a concentration of progastrin of at least 3 pM in said biological sample is indicative of the presence of a cancer which is not responsive to treatment with an immune checkpoint inhibitor in said subject.

2. The method of claim 1, wherein the method comprises the further steps of:
c) determining a reference concentration of progastrin in a reference sample,
d) comparing the concentration of progastrin in said biological sample with said reference concentration of progastrin,
e) determining, from the comparison of step d), whether said patient is responsive or not to treatment with an immune checkpoint inhibitor.

3. The method of any one of claim 1 or 2, wherein the determination of step a) includes:
(i) contacting said sample with a first progastrin-binding antibody which binds to a first epitope of progastrin, and
(ii) contacting said sample with a second progastrin-binding antibody which binds to a second epitope of progastrin.

4. The method of claim 3, wherein the first progastrin-binding antibody binds an epitope within the C-terminus of progastrin.

5. The method of any one of claims 3 or 4, wherein said progastrin-binding antibody is a monoclonal antibody produced by the hybridoma deposited at the CNCM, Institut Pasteur, 25-28 rue du Docteur Roux, 75724 Paris CEDEX 15, France, on 27 December 2016, under reference I-5158.

6. The method of any one of claims 4 to 5, wherein the second progastrin-binding antibody binds an epitope within the N-terminus of progastrin.

7. The method of any one of claims 4 to 6, wherein said second progastrin-binding antibody is:
- a polyclonal antibody binding an epitope within the N-terminus of progastrin or
- a monoclonal antibody comprising a heavy chain comprising the following three CDRs, CDR-H1, CDR-H2 and CDR-H3 of amino acid sequences SEQ ID N°16, 17 and 18, respectively, and a light chain comprising the following three CDRs, CDR-L1, CDR-L2 and CDR-L3 of amino acid sequences SEQ ID N°19, 20 and 21, respectively.

8. The method of any one of claims 1 to 7, wherein the level of progastrin is determined in step a) with an ELISA.

9. The method of any one of claims 1 to 6, wherein said biological sample is contacted with a first antibody, which binds to a first part of progastrin, and with a second antibody, which binds to a second part of progastrin.

10. An immune checkpoint inhibitor for use in treating melanoma said use comprising a prior step of:
a) selecting a patient responsive to immune checkpoint inhibitors using a method according any one of claims 1 to 11.

11. An *in vitro* method for prognosing a melanoma treatment with an immune checkpoint inhibitor in a subject, said method comprising the steps of:
a) contacting a biological sample from said subject with at least one progastrin-binding monoclonal antibody,
wherein the biological sample is blood, plasma or serum,
and wherein the monoclonal antibody is selected in the group consisting of:
- A monoclonal antibody comprising a heavy chain comprising CDR-H1, CDR-H2 and CDR-H3 of amino acid sequences SEQ ID N°4, 5 and 6, respectively, and a light chain comprising CDR-L1, CDR-L2 and CDR-L3 of amino acid sequences SEQ ID N°7, 8 and 9, respectively,
- A monoclonal antibody comprising a heavy chain comprising CDR-H1, CDR-H2 and CDR-H3 of amino acid sequences SEQ ID N°10, 11 and 12, respectively, and a light chain comprising CDR-L1, CDR-L2 and CDR-L3 of amino acid sequences SEQ ID N°13, 14 and 15, respectively,
- A monoclonal antibody comprising a heavy chain comprising CDR-H1, CDR-H2 and CDR-H3 of amino acid sequences SEQ ID N°16, 17 and 18, respectively, and a light chain comprising CDR-L1, CDR-L2 and CDR-L3 of amino acid sequences SEQ ID N°19, 20 and 21, respectively,
- A monoclonal antibody comprising a heavy chain comprising CDR-H1, CDR-H2 and CDR-H3 of amino acid sequences SEQ ID N°22, 23 and 24, respectively, and a light chain comprising CDR-L1, CDR-L2 and CDR-L3 of amino acid sequences SEQ ID N°25, 26 and 27, respectively,
- A monoclonal antibody comprising a heavy chain comprising CDR-H1, CDR-H2 and CDR-H3 of amino acid sequences SEQ ID N°28, 29 and 30, respectively, and a light chain comprising CDR-L1, CDR-L2 and CDR-L3 of amino acid sequences SEQ ID N°31, 32 and 33, respectively,
- A monoclonal antibody comprising a heavy chain comprising CDR-H1, CDR-H2 and CDR-H3 of amino acid sequences SEQ ID N°34, 35 and 36, respectively, and a light chain comprising CDR-L1, CDR-L2 and CDR-L3 of amino acid sequences SEQ ID N°37, 38 and 39, respectively, and
- A monoclonal antibody produced by the hybridoma deposited at the CNCM, Institut Pasteur, 25-28 rue du Docteur Roux, 75724 Paris CEDEX 15, France, on 27 December 2016, under reference I-5158.
and
b) detecting the binding of said progastrin-binding molecule to progastrin in said sample, wherein a concentration of progastrin of at least 3 pM in said biological sample indicates the prognosis is negative.

12. The method of claim 11, wherein the method comprises the further steps of:
a) determining a reference concentration of progastrin in a reference sample,
b) comparing the concentration of progastrin in said biological sample with said reference concentration of progastrin,
c) prognosing, from the comparison of step d), said cancer treatment with an immune checkpoint inhibitor.

## Patentansprüche

1. *In-vitro*-Verfahren zum Auswählen eines Melanompatienten, der für ein Ansprechen auf eine Behandlung mit einem Immun-Checkpoint-Inhibitor empfänglich ist, wobei das Verfahren die Schritte umfasst:
a) Inkontaktbringen einer biologischen Probe von dem Probanden mit mindestens einem Progastrin-bindenden monoklonalen Antikörper,
wobei die biologische Probe Blut, Plasma oder Serum ist,
und wobei der monoklonale Antikörper ausgewählt ist aus der Gruppe bestehend aus:
- einem monoklonalen Antikörper, umfassend eine schwere Kette, umfassend CDR-H1, CDR-H2 und CDR-H3 der Aminosäuresequenzen SEQ ID Nr. 4, 5 bzw. 6, und eine leichte Kette, umfassend CDR-L1, CDR-L2 und CDR-L3 der Aminosäuresequenzen SEQ ID Nr. 7, 8 bzw. 9,
- einem monoklonalen Antikörper, umfassend eine schwere Kette, umfassend CDR-H1, CDR-H2 und CDR-H3 der Aminosäuresequenzen SEQ ID Nr. 10, 11 bzw. 12, und eine leichte Kette, umfassend CDR-L1, CDR-L2 und CDR-L3 der Aminosäuresequenzen SEQ ID Nr. 13, 14 bzw. 15,
- einem monoklonalen Antikörper, umfassend eine schwere Kette, umfassend CDR-H1, CDR-H2 und CDR-H3 der Aminosäuresequenzen SEQ ID Nr. 16, 17 bzw. 18, und eine leichte Kette, umfassend CDR-L1, CDR-L2 und CDR-L3 der Aminosäuresequenzen SEQ ID Nr. 19, 20 bzw. 21,
- einem monoklonalen Antikörper, umfassend eine schwere Kette, umfassend CDR-H1, CDR-H2 und CDR-H3 der Aminosäuresequenzen SEQ ID Nr. 22, 23 bzw. 24, und eine leichte Kette, umfassend CDR-L1, CDR-L2 und CDR-L3 der Aminosäuresequenzen SEQ ID Nr. 25, 26 bzw. 27,
- einem monoklonalen Antikörper, umfassend eine schwere Kette, umfassend CDR-H1, CDR-H2 und CDR-H3 der Aminosäuresequenzen SEQ ID Nr. 28, 29 bzw. 30, und eine leichte Kette, umfassend CDR-L1, CDR-L2 und CDR-L3 der Aminosäuresequenzen SEQ ID Nr. 31, 32 bzw. 33,
- einem monoklonalen Antikörper, umfassend eine schwere Kette, umfassend CDR-H1, CDR-H2 und CDR-H3 der Aminosäuresequenzen SEQ ID Nr. 34, 35 bzw. 36, und eine leichte Kette, umfassend CDR-L1, CDR-L2 und CDR-L3 der Aminosäuresequenzen SEQ ID Nr. 37, 38 bzw. 39, und
- einem monoklonalen Antikörper, der durch das Hybridom produziert wird, das am 27. Dezember 2016 unter der Referenz I-5158 bei der CNCM, Institut Pasteur, 25-28 rue du Docteur Roux, 75724 Paris CEDEX 15, Frankreich, hinterlegt wurde.
und
b) Nachweisen der Bindung des Progastrin-bindenden Moleküls an Progastrin in der Probe, wobei eine Konzentration von Progastrin von mindestens 3 pM in der biologischen Probe das Vorliegen eines Krebses angibt, die nicht auf eine Behandlung mit einem Immun-Checkpoint-Inhibitor bei dem Probanden anspricht.

2. Verfahren nach Anspruch 1, wobei das Verfahren die weiteren Schritte umfasst:
c) Bestimmen einer Referenzkonzentration von Progastrin in einer Referenzprobe,
d) Vergleichen der Konzentration von Progastrin in der biologischen Probe mit der Referenzkonzentration von Progastrin,
e) Bestimmen aus dem Vergleich von Schritt d), ob der Patient auf eine Behandlung mit einem Immun-Checkpoint-Inhibitor anspricht oder nicht.

3. Verfahren nach einem von Anspruch 1 oder 2, wobei die Bestimmung von Schritt a) einschließt:
(i) Inkontaktbringen der Probe mit einem ersten Progastrin-bindenden Antikörper, der an ein erstes Epitop von Progastrin bindet, und
(ii) Inkontaktbringen der Probe mit einem zweiten Progastrin-bindenden Antikörper, der an ein zweites Epitop von Progastrin bindet.

4. Verfahren nach Anspruch 3, wobei der erste Progastrin-bindende Antikörper ein Epitop innerhalb des C-Terminus von Progastrin bindet.

5. Verfahren nach einem der Ansprüche 3 oder 4, wobei der Progastrin-bindende Antikörper ein monoklonaler Antikörper ist, der durch das Hybridom produziert wird, das am 27. Dezember 2016 unter der Referenz I-5158 bei der CNCM, Institut Pasteur, 25-28 rue du Docteur Roux, 75724 Paris CEDEX 15, Frankreich, hinterlegt wurde.

6. Verfahren nach einem der Ansprüche 4 bis 5, wobei der zweite Progastrin-bindende Antikörper ein Epitop innerhalb des N-Terminus von Progastrin bindet.

7. Verfahren nach einem der Ansprüche 4 bis 6, wobei der zweite Progastrin-bindende Antikörper ist:
- ein polyklonaler Antikörper, der ein Epitop innerhalb des N-Terminus von Progastrin bindet, oder
- ein monoklonaler Antikörper, umfassend eine schwere Kette, umfassend die folgenden drei CDR, CDR-H1, CDR-H2 und CDR-H3, der Aminosäuresequenzen SEQ ID Nr. 16, 17 bzw. 18, und eine leichte Kette, umfassend die folgenden drei CDR, CDR-L1, CDR-L2 und CDR-L3, der Aminosäuresequenzen SEQ ID Nr. 19, 20 bzw. 21,

8. Verfahren nach einem der Ansprüche 1 bis 7, wobei der Spiegel von Progastrin in Schritt a) mit einem ELISA bestimmt wird.

9. Verfahren nach einem der Ansprüche 1 bis 6, wobei die biologische Probe mit einem ersten Antikörper, der an einen ersten Teil von Progastrin bindet, und mit einem zweiten Antikörper , der an einen zweiten Teil von Progastrin bindet, in Kontakt gebracht wird.

10. Immun-Checkpoint-Inhibitor zur Verwendung bei der Behandlung von Melanom, wobei die Verwendung eine vorherigen Schritt umfasst:
a) Auswählen eines Patienten, der auf Immun-Checkpoint-Inhibitoren anspricht, unter Verwendung eines Verfahrens nach einem der Ansprüche 1 bis 11.

11. *In-vitro*-Verfahren zum Stellen einer Prognose einer Melanombehandlung mit einem Immun-Checkpoint-Inhibitor bei einem Probanden, wobei das Verfahren die Schritte umfasst:
a) Inkontaktbringen einer biologischen Probe von dem Probanden mit mindestens einem Progastrin-bindenden monoklonalen Antikörper,
wobei die biologische Probe Blut, Plasma oder Serum ist,
und wobei der monoklonale Antikörper ausgewählt ist aus der Gruppe bestehend aus:
- einem monoklonalen Antikörper, umfassend eine schwere Kette, umfassend CDR-H1, CDR-H2 und CDR-H3 der Aminosäuresequenzen SEQ ID Nr. 4, 5 bzw. 6, und eine leichte Kette, umfassend CDR-L1, CDR-L2 und CDR-L3 der Aminosäuresequenzen SEQ ID Nr. 7, 8 bzw. 9,
- einem monoklonalen Antikörper, umfassend eine schwere Kette, umfassend CDR-H1, CDR-H2 und CDR-H3 der Aminosäuresequenzen SEQ ID Nr. 10, 11 bzw. 12, und eine leichte Kette, umfassend CDR-L1, CDR-L2 und CDR-L3 der Aminosäuresequenzen SEQ ID Nr. 13, 14 bzw. 15,
- einem monoklonalen Antikörper, umfassend eine schwere Kette, umfassend CDR-H1, CDR-H2 und CDR-H3 der Aminosäuresequenzen SEQ ID Nr. 16, 17 bzw. 18, und eine leichte Kette, umfassend CDR-L1, CDR-L2 und CDR-L3 der Aminosäuresequenzen SEQ ID Nr. 19, 20 bzw. 21,
- einem monoklonalen Antikörper, umfassend eine schwere Kette, umfassend CDR-H1, CDR-H2 und CDR-H3 der Aminosäuresequenzen SEQ ID Nr. 22, 23 bzw. 24, und eine leichte Kette, umfassend CDR-L1, CDR-L2 und CDR-L3 der Aminosäuresequenzen SEQ ID Nr. 25, 26 bzw. 27,
- einem monoklonalen Antikörper, umfassend eine schwere Kette, umfassend CDR-H1, CDR-H2 und CDR-H3 der Aminosäuresequenzen SEQ ID Nr. 28, 29 bzw. 30, und eine leichte Kette, umfassend CDR-L1, CDR-L2 und CDR-L3 der Aminosäuresequenzen SEQ ID Nr. 31, 32 bzw. 33,
- einem monoklonalen Antikörper, umfassend eine schwere Kette, umfassend CDR-H1, CDR-H2 und CDR-H3 der Aminosäuresequenzen SEQ ID Nr. 34, 35 bzw. 36, und eine leichte Kette, umfassend CDR-L1, CDR-L2 und CDR-L3 der Aminosäuresequenzen SEQ ID Nr. 37, 38 bzw. 39, und
- einem monoklonalen Antikörper, der durch das Hybridom produziert wird, das am 27. Dezember 2016 unter der Referenz I-5158 bei der CNCM, Institut Pasteur, 25-28 rue du Docteur Roux, 75724 Paris CEDEX 15, Frankreich, hinterlegt wurde.
und
b) Nachweisen der Bindung des Progastrin-bindenden Moleküls an Progastrin in der Probe, wobei eine Konzentration von Progastrin von mindestens 3 pM in der biologischen Probe angibt, dass die Prognose negativ ist.

12. Verfahren nach Anspruch 11, wobei das Verfahren die weiteren Schritte umfasst:
a) Bestimmen einer Referenzkonzentration von Progastrin in einer Referenzprobe,
b) Vergleichen der Konzentration von Progastrin in der biologischen Probe mit der Referenzkonzentration von Progastrin,
c) Stellen einer Prognose der Krebsbehandlung mit einem Immun-Checkpoint-Inhibitor aus dem Vergleich von Schritt d).

## Revendications

1. Procédé *in vitro* permettant de sélectionner un patient atteint d'un mélanome susceptible de répondre à un traitement par un inhibiteur de point de contrôle immunitaire, ledit procédé comprenant les étapes suivantes de :
a) mise en contact un échantillon biologique provenant dudit sujet avec au moins un anticorps monoclonal se liant à la progastrine,
l'échantillon biologique étant du sang, du plasma ou du sérum,
et dans laquelle l'anticorps monoclonal est choisi parmi le groupe constitué de :
- un anticorps monoclonal comprenant une chaîne lourde comprenant les CDR-H1, CDR-H2 et CDR-H3 des séquences d'acides aminés SEQ ID N° 4, 5 et 6, respectivement, et une chaîne légère comprenant les CDR-L1, CDR-L2 et CDR-L3 des séquences d'acides aminés SEQ ID N° 7, 8 et 9, respectivement,
- Un anticorps monoclonal comprenant une chaîne lourde comprenant les CDR-H1, CDR-H2 et CDR-H3 des séquences d'acides aminés SEQ ID N° 10, 11 et 12, respectivement, et une chaîne légère comprenant les CDR-L1, CDR-L2 et CDR-L3 des séquences d'acides aminés SEQ ID N° 13, 14 et 15, respectivement,
- Anticorps monoclonal comprenant une chaîne lourde comprenant les CDR-H1, CDR-H2 et CDR-H3 des séquences d'acides aminés SEQ ID N° 16, 17 et 18, respectivement, et une chaîne légère comprenant les CDR-L1, CDR-L2 et CDR-L3 des séquences d'acides aminés SEQ ID N° 19, 20 et 21, respectivement,
- Anticorps monoclonal comprenant une chaîne lourde comprenant les CDR-H1, CDR-H2 et CDR-H3 des séquences d'acides aminés SEQ ID N° 22, 23 et 24, respectivement, et une chaîne légère comprenant les CDR-L1, CDR-L2 et CDR-L3 des séquences d'acides aminés SEQ ID N° 25, 26 et 27, respectivement,
- Anticorps monoclonal comprenant une chaîne lourde comprenant les CDR-H1, CDR-H2 et CDR-H3 des séquences d'acides aminés SEQ ID N° 28, 29 et 30, respectivement, et une chaîne légère comprenant les CDR-L1, CDR-L2 et CDR-L3 des séquences d'acides aminés SEQ ID N° 31, 32 et 33, respectivement,
- Un anticorps monoclonal comprenant une chaîne lourde comprenant les CDR-H1, CDR-H2 et CDR-H3 des séquences d'acides aminés SEQ ID N°34, 35 et 36, respectivement, et une chaîne légère comprenant les CDR-L1, CDR-L2 et CDR-L3 des séquences d'acides aminés SEQ ID N°37, 38 et 39, respectivement, et
- Un anticorps monoclonal produit par l'hybridome déposé à la CNCM, Institut Pasteur, 25-28 rue du Docteur Roux, 75724 Paris CEDEX 15, France, le 27 décembre 2016, sous la référence I-5158.
et
b) détection de la liaison de ladite molécule se liant à la progastrine à la progastrine dans ledit échantillon, dans laquelle une concentration en progastrine d'au moins 3 pM dans ledit échantillon biologique est indicative de la présence d'un cancer qui ne répond pas à un traitement par un inhibiteur de point de contrôle immunitaire chez ledit sujet.

2. Procédé selon la revendication 1, dans lequel le procédé comprend les étapes supplémentaires consistant à :
c) déterminer une concentration de référence de progastrine dans un échantillon de référence,
d) comparer la concentration en progastrine dans ledit échantillon biologique à ladite concentration de référence en progastrine,
e) déterminer, à partir de la comparaison de l'étape d), si ledit patient répond ou non à un traitement par un inhibiteur de point de contrôle immunitaire.

3. Procédé selon l'une quelconque des revendications 1 ou 2, dans lequel la détermination de l'étape a) comprend :
(i) la mise en contact dudit échantillon avec un premier anticorps se liant à la progastrine, qui se lie à un premier épitope de la progastrine, et
(ii) la mise en contact dudit échantillon avec un deuxième anticorps de liaison à la progastrine qui se lie à un second épitope de la progastrine.

4. Procédé selon la revendication 3, dans lequel le premier anticorps se liant à la progastrine se lie à un épitope dans l'extrémité C-terminale de la progastrine.

5. Procédé selon l'une quelconque des revendications 3 ou 4, dans lequel ledit anticorps de liaison à la progastrine est un anticorps monoclonal produit par l'hybridome déposé à la CNCM, Institut Pasteur, 25-28 rue du Docteur Roux, 75724 Paris CEDEX 15, France, le 27 décembre 2016, sous la référence I-5158.

6. Procédé selon l'une quelconque des revendications 4 à 5, dans lequel le deuxième anticorps se liant à la progastrine se lie à un épitope dans l'extrémité N-terminale de la progastrine.

7. Procédé selon l'une quelconque des revendications 4 à 6, dans lequel ledit deuxième anticorps se liant à la progastrine est :
- un anticorps polyclonal se liant à un épitope situé au sein de l'extrémité N-terminale de la progastrine ou
- un anticorps monoclonal comprenant une chaîne lourde comportant les trois CDR suivants, CDR-H1, CDR-H2 et CDR-H3, correspondant respectivement aux séquences d'acides aminés SEQ ID N° 16, 17 et 18, et une chaîne légère comportant les trois CDR suivants, CDR-L1, CDR-L2 et CDR-L3 des séquences d'acides aminés SEQ ID N° 19, 20 et 21, respectivement.

8. Procédé selon l'une quelconque des revendications 1 à 7, dans lequel le taux de progastrine est déterminé à l'étape a) par un test ELISA.

9. Procédé selon l'une quelconque des revendications 1 à 6, dans lequel ledit échantillon biologique est mis en contact avec un premier anticorps, qui se lie à une première partie de la progastrine, et avec un deuxième anticorps, qui se lie à une deuxième partie de la progastrine.

10. Inhibiteur de point de contrôle immunitaire pour son utilisation dans le traitement du mélanome, ladite utilisation comprenant une étape préalable consistant à :
a) sélectionner un patient répondant aux inhibiteurs de points de contrôle immunitaires à l'aide d'un procédé selon l'une quelconque des revendications 1 à 11.

11. Procédé *in vitro* permettant de prédire l'efficacité d'un traitement du mélanome par un inhibiteur de point de contrôle immunitaire chez un sujet, ledit procédé comprenant les étapes consistant à :
a) mettre en contact un échantillon biologique provenant dudit sujet avec au moins un anticorps monoclonal se liant à la progastrine,
dans laquelle l'échantillon biologique est du sang, du plasma ou du sérum,
et dans laquelle l'anticorps monoclonal est choisi dans le groupe constitué par :
- un anticorps monoclonal comprenant une chaîne lourde comprenant les CDR-H1, CDR-H2 et CDR-H3 des séquences d'acides aminés SEQ ID N° 4, 5 et 6, respectivemen , et une chaîne légère comprenant les CDR-L1, CDR-L2 et CDR-L3 des séquences d'acides aminés SEQ ID N° 7, 8 et 9, respectivement,
- Anticorps monoclonal comprenant une chaîne lourde comprenant les CDR-H1, CDR-H2 et CDR-H3 des séquences d'acides aminés SEQ ID N° 10, 11 et 12, respectivement, et une chaîne légère comprenant les CDR-L1, CDR-L2 et CDR-L3 des séquences d'acides aminés SEQ ID N° 13, 14 et 15, respectivement,
- Anticorps monoclonal comprenant une chaîne lourde comprenant les CDR-H1, CDR-H2 et CDR-H3 des séquences d'acides aminés SEQ ID N° 16, 17 et 18, respectivement, et une chaîne légère comprenant les CDR-L1, CDR-L2 et CDR-L3 des séquences d'acides aminés SEQ ID N° 19, 20 et 21, respectivement,
- Anticorps monoclonal comprenant une chaîne lourde comprenant les CDR-H1, CDR-H2 et CDR-H3 des séquences d'acides aminés SEQ ID N° 22, 23 et 24, respectivement, et une chaîne légère comprenant les CDR-L1, CDR-L2 et CDR-L3 des séquences d'acides aminés SEQ ID N° 25, 26 et 27, respectivement,
- Anticorps monoclonal comprenant une chaîne lourde comprenant les CDR-H1, CDR-H2 et CDR-H3 des séquences d'acides aminés SEQ ID N° 28, 29 et 30, respectivement, et une chaîne légère comprenant les CDR-L1, CDR-L2 et CDR-L3 des séquences d'acides aminés SEQ ID N° 31, 32 et 33, respectivement,
- Un anticorps monoclonal comprenant une chaîne lourde comprenant les CDR-H1, CDR-H2 et CDR-H3 des séquences d'acides aminés SEQ ID N° 34, 35 et 36, respectivement, et une chaîne légère comprenant les CDR-L1, CDR-L2 et CDR-L3 des séquences d'acides aminés SEQ ID N° 37, 38 et 39, respectivement, et
- un anticorps monoclonal produit par l'hybridome déposé au CNCM, Institut Pasteur, 25-28 rue du Docteur Roux, 75724 Paris CEDEX 15, France, le 27 décembre 2016, sous la référence I-5158.
et
b) la détection de la liaison de ladite molécule de liaison à la progastrine à la progastrine dans ledit échantillon, dans laquelle une concentration en progastrine d'au moins 3 pM dans ledit échantillon biologique indique que le pronostic est négatif.

12. Procédé selon la revendication 11, dans lequel le procédé comprend les étapes supplémentaires consistant à :
a) déterminer une concentration de référence de progastrine dans un échantillon de référence,
b) comparer la concentration en progastrine dans ledit échantillon biologique à ladite concentration de référence en progastrine,
c) établir un pronostic concernant le traitement dudit cancer par un inhibiteur de point de contrôle immunitaire à partir de la comparaison de l'étape d).
